# EUROPEAN PATENT APPLICATION

(11) **EP 2 172 458 A1**
(43) Date of publication of application: **07.04.2010**
(21) Application number: 08777693.6
(22) Date of filing: 23.06.2008
(51) Int. Cl.: C07D 231/20, A61K 31/4152, A61K 31/4155, A61K 31/454, A61P 7/02, A61P 9/10, A61P 11/00, A61P 13/12, C07D 231/22, C07D 401/06, C07D 405/12

(54) **PYRAZOLONE DERIVATIVE**

(30) Priority: 27.06.2007 JP 2007169523
(71) Applicant: Kowa Company, Ltd., Naka-ku Nagoya-shi, Aichi-ken 460-8625 (JP)
(72) Inventor: GOMI, Noriaki, Higashimurayama-shi Tokyo 189-0022 (JP); INA, Shinji, Higashimurayama-shi Tokyo 189-0022 (JP); YAMANA, Kenjirou, Higashimurayama-shi Tokyo 189-0022 (JP); KANEKO, Yoshio, Higashimurayama-shi Tokyo 189-0022 (JP)
(74) Representative: Cohausz & Florack
(86) International application number: PCT/JP2008/061787
(87) International publication number: WO 2009/001949

(57) **Abstract**

A pyrazolone derivative represented by formula (I) below: 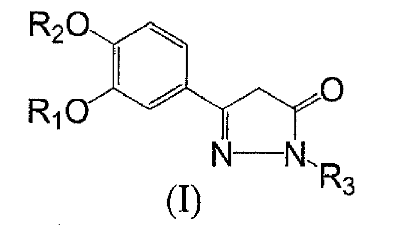 wherein R₁ to R₃ are the same as defined in claims; or an optical isomer, a pharmaceutically acceptable salt thereof, or a hydrate or solvate thereof is provided. The novel pyrazolone derivative according to the present invention has a PAI-1 production inhibitory activity, a tissue fibrosis inhibitory activity, and a fibrolytic activity, and is effective for preventing and/or treating tissue fibrotic diseases (lung fibrosis, kidney fibrosis, etc.) and diseases of which a pathological thrombus becomes the cause, such as ischemic cardiac diseases (cardiac infarction and angina pectoris), atrial thrombus, lung embolism, deep thrombophlebitis, disseminated intravascular clotting, ischemic brain diseases (brain infarction, brain hemorrhage), and arterial sclerosis. In addition, a pharmaceutical agent for preventing and/or treating the disease conditions or the symptoms mediated by plasminogen activator inhibitor-1, comprising the novel pyrazolone derivative according to the present invention is also provided.

## Description

### FIELD OF THE INVENTION

The present invention relates to a pyrazolone derivative having a plasminogen activator inhibitor-1 (hereinafter abbreviated as PAT-1) production inhibitory activity.

### BACKGROUND ART

Thrombogenesis is an important biological reaction forming hemostatic plugs to stop bleeding in normal states. However, abnormal thrombogenesis is considered to be a cause of ischemic diseases which is considered to be one of the major causes of death in Japan and Western countries. There are quite a few diseases of which a pathological thrombus becomes the cause; ischemic cardiac diseases (cardiac infarction, angina pectoris), atrial thrombus, lung embolism, venous thromboembolism, disseminated intravascular clotting, ischemic brain diseases (brain infarction, brain hemorrhage), arterial sclerosis, etc. are reported. Several drugs are currently used for these pathological blood clots. As typical examples, an antiplatelet agent, a hemostatic factor inhibiting agent, a vitamin K inhibitor, and a fibrinolytic agent are exemplified.

Activation of the fibrinolytic system is initiated via activation of plasminogen to plasmin by t-PA and urokinase which are plasminogen activators, and thrombolysis progresses as the generated plasmin decomposes fibrin, a component of thrombus. In this system, the physiological control factor against the plasminogen activators is PAI-1 which is released from vascular endothelial cells and activated platelets. PAI-1 is a glycoprotein with a molecular weight of approximately 50 kDa composed of 379 amino acids without an intramolecular S-S bond. PAI-1 inhibits t-PA activity by binding with the enzyme-activity center of t-PA at a 1:1 molar ratio. In normal conditions, as the regulatory substances of the fibrinolytic system balance with each other, the flow of blood runs smoothly in the blood vessels without hemorrhage and thrombus. However, it is thought that generation of t-PA is reduced in pathological thrombosis and vascular endothelial cells while secretion of PAI-1 is enhanced. Therefore, inhibition of PAI-1 which increases in pathological thrombus is considered to show a therapeutic effect.

On the other hand, thrombosis is not the only pathological condition in which fibrin is involved, but in tissue fibrosis, it is known that fibrin clots are formed rigidly. Fibrosis is a pathological state observed in a number of organs such as the lungs, kidney, liver, skin, central nervous system, and blood vessels, but even its pathological cause has not been elucidated, not to mention its treatment. There is a report regarding lung fibrosis induced by bleomycin, using an animal model of fibrosis. It is reported that an increase in the hydroxyproline amount, a marker of fibrosis, is suppressed using PAI-1 knockout mice, and that, conversely the generation of hydroxyproline increases in mice which overexpress PAI-1, relative to controls (J. Clin. Invest., 97, 232 (1996)). It is also reported that administration of uPA which increases after inhibition of PAI-1 can lyse the formed fibra (Clin. Invest. Med., 17: 69-76, (1994)). From the above, it is thought that the inhibition of PAI-1 shows a therapeutic effect on fibrosis.

Presently, compounds showing inhibition of PAI-1 are publicly known (Kokais (Japanese unexamined patent publications) No.7-149642, No.7-149643, No.7-165573, No.7-165574, No.10-287622, No.2003-89687, No.2004-203793, Europatent publication No.0563798, WO95/32190, WO03/000684, etc.), but these compounds are not being used clinically, and the development of more effective compounds is anticipated.

WO06/062212 describes that a compound having the arylidene pyrazolone structure represented by the formula below (VI): 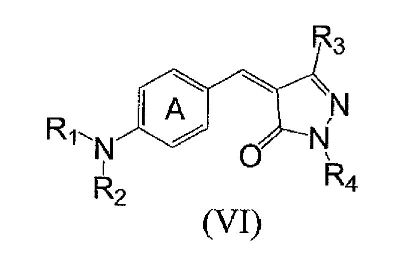 [wherein ring A represents a benzene ring which may have an optional substituent; R₁ and R₂ each independently represent a hydrogen atom, an alkyl group which may be substituted, an allyl group, an aryl group which may be substituted, or a cycloalkyl group which may be substituted; R₃ represents a hydrogen atom, an alkyl group which may be substituted, or an aryl group which may be substituted, etc.; and R₄ represents an alkyl group which may be substituted or an aryl group which may be substituted] is useful for a dye.

The compound of above formula (VI), which is **characterized in that** the 4th position of the pyrazolone ring is substituted by arylidene, is quite different in structure from the compound of the present invention. In addition, it was not described or suggested at all that the compound of the above formula (VI) per se shows a PAI-1 production inhibitory activity.

Alternatively, US Patent No.6280919 describes a compound having the aryloxypyrazolone structure represented by the following formula (VII): 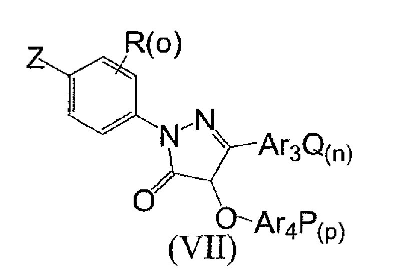 [wherein Z represents a hydrogen atom, or an alkyl group, etc.; and Ar₃ and Ar₄ individually represent an aryl group]. The compound of the above formula (VII), which is **characterized in that** it has an aryloxy group at the 4th position of the pyrazolone ring, is quite different in structure from the compound in the present invention. In addition, it was not described or suggested at all that the compound of the above formula (VII) per se shows a PAI-1 production inhibitory activity. Furthermore, WO98/051268 describes a compound having a pyrazoline-4,5-dione structure represented by the formula below (VIII): 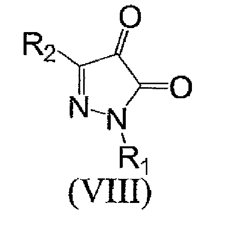 [wherein R₁ represents a hydroxyl group, a carboxyl group, or a C₁ to C₄ alkoxy group, etc.; and R₂ represents a phenyl group which may be substituted with a halogen atom, a nitro group, or a trifluoromethyl group, etc.]. The compound of the above formula (VIII), which is **characterized in that** the 4th position of the pyrazolone ring is substituted with an oxo group, is quite different in structure from the compound of the present invention. In addition, it was not described or suggested at all that the compound of the above formula (VIII) per se shows a PAI-1 production inhibitory activity.

Moreover, DE patent publication No.3941240 describes an herbicide represented by the formula below (IX): 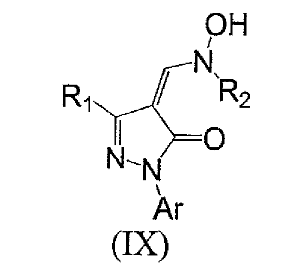 [wherein R₁ represents an aryl group which may be substituted with alkyl, or cycloalkyl, etc.; R₂ represents hydrogen, alkyl, or cycloalkyl, etc.; and Ar represents an aryl group, etc which may be substituted]. The compound of the above formula (IX), which is **characterized in that** the pyrazolone ring has an alkylidene substituent at the 4th position, is quite different in structure from the compound of the present invention. In addition, it was not described or suggested at all that the compound of the above formula (IX) per se shows a PAI-1 production inhibitory activity.

### DISCLOSURE OF THE INVENTION

The object of the present invention is to discover a compound having a PAI-1 production inhibitory activity, a tissue fibrosis inhibitory activity, and a fibrolytic acitivity, and to provide a medical drug for preventing and/or treating diseases, of which a pathological thrombus becomes the cause, such as ischemic cardiac diseases (cardiac infarction, angina pectoris), atrial thrombus, lung embolism, deep thrombophlebitis, disseminated intravascular clotting, ischemic brain diseases (brain infarction, brain hemorrhage), and arteriosclerosis.

The present inventors searched for a compound having a PAI-1 production inhibitory activity, and discovered that the pyrazolone derivative of the present invention has strong a PAI-1 production inhibitory activity, a tissue fibrosis inhibitory activity, and a fibrolytic activity, and thereby completed the present invention. Namely, the present invention relates to a 5-phenyl-3-pyrazolone derivative represented by the following formula (I): 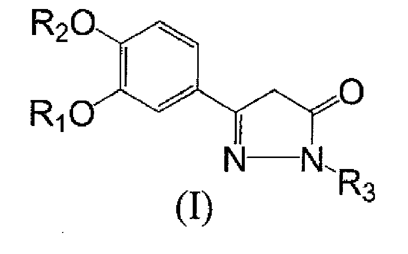 [wherein R₁ represents a C₁ to C₈ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or fused polycyclic aryl group; or a monocyclic or fused polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more of said substituents, or an indanyl group; R₂ represents a C₁ to C₄ alkyl group, a C₁ to C₄ haloalkyl group, or a C₃ to C₇ cycloalkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more of an alkyl group on the nitrogen atom; an alkoxy group; an alkoxycarbonyl group; an acyl group; a monocyclic or fused polycyclic aryl group; or a monocyclic or fused polycyclic hetero ring having one or more hetero atoms); and R₃ represents a hydrogen atom, a C₁ to C₅ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or fused polycyclic aryl group; or a monocyclic or fused polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more substituents (a methyl group; a hydroxyl group; or a carboxyl group), a monocyclic or fused polycyclic aryl group which may have one or more substituents (a methyl group; a hydroxyl group; or a carboxyl group), or a monocyclic or fused polycyclic hetero aryl group having one or more hetero atoms; or an optical isomer, a pharmaceutically acceptable salt thereof, or a hydrate or solvate thereof].

The compound of the present invention has an excellent PAI-1 production inhibitory activity, and is effective, by the fibrinolytic activity, as a medical drug for preventing and/or treating tissue fibrotic diseases such as lung fibrosis and kidney fibrosis, and diseases of which a pathological thrombus becomes the cause, such as ischemic cardiac diseases (cardiac infarction, angina pectoris), atrial thrombus, lung embolism, and deep thrombophlebitis.

### BEST MODE FOR CARRYING OUT THE INVENTION

R₁ of the above formula (I) represents a C₁ to C₈ alkyl group which may have one or more substituents, a C₃ to C₇ cycloalkyl group which may have one or more substituents, a 3 to 7-membered monocyclic nonaromatic hetero ring with one or more hetero atoms which may have one or more substituents, or an indanyl group.

Rearding the "substituents" in the "C₁ to C₈ alkyl which may have one or more substituents", the "C₃ to C₇ cycloalkyl group which may have one or more substituents", and the "3 to 7-membered monocyclic nonaromatic hetero ring with one or more hetero atoms which may have one or more substituents" which are represented by R₁, a halogen atom (for example, fluorine; chlorine; bromine; iodine), a hydroxyl group, a nitro group, a cyano group, an amino group which may have one or more C₁ to C₄ alkyl or benzyl groups on the nitrogen atom, a carboxyl group, a C₁ to C₆ alkyl group, a C₃ to C₇ cycloalkyl group which may have a substituent (a phenyl or a C₁ to C₆ alkyl group), a C₁ to C₆ haloalkyl group, a carbamoyl group which may have a C₁ to C₄ alkyl group as a substituent on the nitrogen atom, a C₁ to C₆ alkoxy group, a C₁ to C₆ alkoxycarbonyl group, a C₁ to C₆ acyl group, a 6 to 10-membered monocyclic or fused polycyclic aryl group, or a monocyclic or fused polycyclic hetero ring having one or more hetero atoms (for example, a 3 to 10-membered monocyclic or fused polycyclic nonaromatic hetero ring with one or more hetero atoms, and a 5 to 10-membered monocyclic or fused polycyclic hetero aryl group), etc. are exemplified.

Regarding R₁, preferably, a C₁ to C₈ alkyl group, a C₁ to C₅ alkyl group having one or more phenyl or furyl groups as substituents, a C₁ to C₅ alkyl group with one or more C₃ to C₇ cycloalkyl groups, as substituents, which may have one or more phenyl or C₁ to C₃ alkyl groups as substituents, a C₄ to C₆ cycloalkyl group which may have one or more substituents (a methyl group; hydroxyl group; a carboxyl group; etc.), a 5 to 6-membered nonaromatic hetero ring with one or more hetero atoms which may have one or more substituents (a methyl group; hydroxyl group; a carboxyl group; etc.), or an indanyl group are exemplified. As specifically preferable groups for R₁, a methyl group, an ethyl group, a propyl group, a butyl group, an isobutyl group, a pentyl group, a 3-pentyl group, a neopentyl group, a 2-ethylbutyl group, a cyclopropylmethyl group, a cyclobutylmethyl group, a cyclopentylmethyl group, a cyclohexylmethyl group, a benzyl group, a phenethyl group, a 3-phenylpropyl group, a 4-phenylbutyl group, a (1-methylcyclopropyl) methyl group, a (1-phenylcyclopropyl) methyl group, a 2-furylmethyl group, a 2-furylethyl group, a 3-(2-furyl) propyl group, a cyclopentyl group, a 3-tetrahydrofuryl group, or a 2-indanyl group are preferable, and above all, a methyl group, 3-pentyl group, a cyclopentyl group, 3-tetrahydrofuryl group, a cyclopropylmethyl group, and an 2-indanyl group are exemplified.

R₂ represents a C₁ to C₄ alkyl group, a C₁ to C₄ haloalkyl group, or a C₃ to C₇ cycloalkyl group which may have substituents. Regarding the "substituents" in the "C₃ to C₇ cycloalkyl group which may have one or more substituents" represented by R₂, a halogen atom (for example, fluorine; chlorine; bromine; iodine), a hydroxyl group, a nitro group, a cyano group, an amino group which may have one or more C₁ to C₄ alkyl or benzyl groups on the nitrogen atom, a carboxyl group, a C₁ to C₆ alkyl group, a C₃ to C₇ cycloalkyl group which may have a substituent (a phenyl or a C₁ to C₆ alkyl group), a C₁ to C₆ haloalkyl group, a carbamoyl group which may have a C₁ to C₄ alkyl group, as a substituent, on the nitrogen atom, a C₁ to C₆ alkoxy group, a C₁ to C₆ alkoxycarbonyl group, a C₁ to C₆ acyl group, a 6 to 10-membered monocyclic or fused polycyclic aryl group, or a monocyclic or fused d polycyclic hetero ring having one or more hetero atoms (for example, a 3 to 10-membered monocyclic or fused polycyclic nonaromatic hetero ring with one or more hetero atoms, or a 5 to 10-membered monocyclic or fused polycyclic heteroaryl group with one or more hetero atoms) etc. are exemplified.

Regarding R₂, preferably, a methyl group, an ethyl group, a propyl group, a butyl group, an isobutyl group, a trifluoromethyl group, a difluoromethyl group, a fluoromethyl group, a cyclobutyl group, a cyclopentyl group, a cyclohexyl group, a C₄ to C₆ cycloalkyl group having one or more substituents (a methyl group, a hydroxyl group, a hydroxycarbonyl group) are exemplified, and above all, a methyl group, an ethyl group, a trifluoromethyl group, a difluoromethyl group, and a cyclopentyl group are exemplified as the specifically preferable groups.

R₃ represents a hydrogen atom, a C₁ to C₅ alkyl group which may have one or more substituents, a C₃ to C₇ cycloalkyl group which may have one or more substituents, a 3 to 7-membered monocyclic nonaromatic hetero ring with one or more hetero atoms which may have one or more substituents, or a monocyclic or fused polycyclic aryl group (a 6 to 10-membered monocyclic or fused polycyclic aryl group), or a monocyclic or fused polycyclic hetero aryl group having one or more hetero atoms (a 5 to 10-membered monocyclic or fused polycyclic hetero aryl group).

Regarding the "substituents" in the "C₁ to C₅ alkyl group which may have one or more substituents", and the "C₃ to C₇ cycloalkyl group which may have one or more substituents", which are represented by R₃, a halogen atom (for example, fluorine; chlorine; bromine; iodine), a hydroxyl group, a nitro group, a cyano group, an amino group which may have one or more C₁ to C₄ alkyl or benzyl groups on the nitrogen atom, a carboxyl group, a C₁ to C₆ alkyl group, a C₃ to C₇ cycloalkyl group which may have a substituent (a phenyl or a C₁ to C₆ alkyl group), a C₁ to C₆ haloalkyl group, a carbamoyl group which may have a C₁ to C₄ alkyl group, as a substituent, on the nitrogen atom, a C₁ to C₆ alkoxy group, a C₁ to C₆ alkoxycarbonyl group, a C₁ to C₆ acyl group, a 6 to 10-membered monocyclic or fused polycyclic aryl group which may have one or more substituents (a methyl group; a hydroxyl group; a carboxyl group), or a monocyclic or fused polycyclic hetero ring having one or more hetero atoms (for example, a 3 to 10-membered monocyclic or fused polycyclic nonaromatic hetero ring with one or more hetero atoms, or a 5 to 10-membered monocyclic or fused polycyclic heteroaryl group with one or more hetero atoms) etc. are exemplified.

Regarding the preferable substituent onto the C₁ to C₅ alkyl group as R₃, a C₃ to C₇ cycloalkyl group, a dibenzylamino group, a carboxyl group, a 1-methylpiperidin-3-yl group, a phenyl group, a naphthyl group, a pyridyl group, a furyl group, a thiazolyl group, a quinolyl group, etc. are exemplified.

Regarding the C₁ to C₅ alkyl group, for R₃, which may have substituents, a benzyl group, a phenethyl group, a 3-phenylpropyl group, a 4-phenylbutyl group, a 5-phenylpentyl group, a pyridylmethyl group, a furylmethyl group, a thiazolylmethyl group, a 1-naphthylmethyl group, a 4-quinolylmethyl group, a cyclopropylmethyl group, a cyclobutylmethyl group, a cyclopentylmethyl group, a cyclohexylmethyl group, a (1-methylcyclopropyl)methyl group, a (1-phenylcyclopropyl)methyl group, a 2-furylmethyl group, a 2-furylethyl group, a 2-(dibenzylamino)ethyl group, a hydroxycarbonylmethyl group, a (1-methylpiperidine-3-yl)methyl group, etc. are exemplified.

Furthermore, regarding R₃, a C₃ to C₇ cycloalkyl group (a cyclopropyl group, a cyclobutyl group, a cyclopentyl group, a cyclohexyl group, a cycloheptyl group, etc.), a 6 to 10-membered monocyclic or fused polycyclic aryl group (a phenyl group, a naphthyl group, 4-hydroxycarbonylphenyl group) which may have one or more substituents (a methyl group; a hydroxyl group; a carboxyl group), or a 5 to 10-membered monocyclic or fused polycyclic heteroaryl group with one or more hetero atoms (a pyridyl group, a thiazolyl group, a furyl group, a thienyl group, a quinolyl group, an oxiranyl group, an aziridinyl group, a tetrahydrofuryl group, etc.) are exemplified.

Regarding R₃, preferably, a hydrogen atom, a C₁ to C₃ alkyl group which may have a substituent (a C₁ to C₃ alkyl group; a carboxyl group; an amino group which may have one or more C₁ to C₄ alkyl or benzyl groups on the nitrogen atom; a piperidine group which may have one or more C₁ to C₄ alkyl or benzyl groups on the nitrogen atom; a phenyl group), a C₄ to C₆ cycloalkyl group (a cyclobutyl group, a cyclopentyl group, a cyclohexyl group) which may have one or more substituents (a methyl group; hydroxyl group; a carboxyl group), a 6 to 10-membered monocyclic or fused polycyclic aryl group (a phenyl group; a naphthyl group, 4-hydroxycarbonylphenyl group) which may have one or more substituents (a methyl group; a hydroxyl group; a carboxyl group), a 5 to 10-membered monocyclic or fused polycyclic heteroaryl group with one or more hetero atoms (a pyridyl group, a thiazolyl group, a furyl group, a thienyl group, a quinolyl group, an oxiranyl group, an aziridinyl group, a tetrahydrofuryl group, etc.), and a C₁ to C₂ alkyl having a substituent (a phenyl group; a pyridyl group; a piperazine group; a piperidinyl group; an amino group which may have one or more C₁ to C₄ alkyl or benzyl groups on the nitrogen atom, etc.) are exemplified, and a hydrogen atom, a methyl group, an ethyl group, a cyclopentyl group, a phenyl group, a benzyl group, a phenethyl group, a 4-hydroxycarbonylphenyl group, a 2-(dibenzylamino)ethyl group, a hydroxycarbonylmethyl group, a (1-methylpiperidin-3-yl)methyl group are specifically preferable.

When further details of the present invention are described, regarding the above formula (I),
R₁ represents a C₁ to C₈ alkyl group (a methyl group; an ethyl group; a propyl group; an isopropyl group; a butyl group; a sec-butyl group; a tert-butyl group; a pentyl group; a hexyl group; an isopentyl group; a neopentyl group; a 1-methylpropyl group; an isohexyl group; a 1,1-dimethylbutyl group; a 2,2-dimethylbutyl group; a 3,3-dimethylbutyl group; a heptyl group; or an octyl group)
which may have one or more substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)},
a C₃ to C₇ cycloalkyl group (a cyclopropyl group; a cyclobutyl group; a cyclopentyl group; a cyclohexyl group; a cycloheptyl group)
which may have one or more substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)},
a 3 to 7-membered monocyclic nonaromatic hetero ring with one or more hetero atoms (an oxiranyl group, an aziridinyl group, an oxetanyl group, a thietanyl group, a thiolanyl group, a thiomorpholinyl group, a pyrrolidinyl group, a pyrrolinyl group, an imidazolidinyl group, an imidazolinyl group, a tetrahydrofuryl group, a pyrazolidinyl group, a pyrazolinyl group, a pyranyl group, a tetrahydropyranyl group, a piperazinyl group, a morpholinyl group, a piperidinyl group, etc.),
which may have one or more substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)}, or an indanyl group;
R₂ represents a C₃ to C₇ cycloalkyl group (a cyclopropyl group; a cyclobutyl group; a cyclopentyl group; a cyclohexyl group; a cycloheptyl group)
which may have one or more methyl, ethyl, propyl, or butyl groups, or substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)}, and
R₃ represents a C₁ to C₅ alkyl group (a methyl group; an ethyl group; a propyl group; a butyl group; a pentyl group, etc.)
which may have one or more hydrogen atoms or substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)}, or
a C₃ to C₇ cycloalkyl group (a cyclopropyl group; a cyclobutyl group; a cyclopentyl group; a cyclohexyl group; a cycloheptyl group)
which may have one or more substituents {fluorine; chlorine; bromine; iodine; a hydroxyl group; a nitro group; a cyano group; an amino group; a methylamino group; an ethylamino group; a propylamino group; an isopropylamino group; a butylamino group; a sec-butylamino group; a tert-butylamino group; a benzylamino group; a dimethylamino group; a diethylamino group; a dipropylamino group; a diisopropylamino group; a dibutylamino group; a di-sec-butylamino group; a di-tert-butylamino group; a dibenzylamino group; an N-ethyl-N-methylamino group; an N-methyl-N-propylamino group; an N-isopropyl-N-methylamino group; an N-butyl-N-methylamino group; an N-sec-butyl-N-methylamino group; an N-tert-butyl-N-methylamino group; an N-benzyl-N-methylamino group; an N-ethyl-N-propylamino group; an N-ethyl-N-isopropylamino group; an N-butyl-N-ethylamino group; an N-sec-butyl-N-ethylamino group; an N-tert-butyl-N-ethylamino group; an N-benzyl-N-ethylamino group; an N-isopropyl-N-propylamino group; an N-butyl-N-propylamino group; an N-sec-butyl-N-propylamino group; an N-tert-butyl-N-propylamino group; an N-benzyl-N-propylamino group; an N-butyl-N-isopropylamino group; an N-sec-butyl-N-isopropylamino group; an N-tert-butyl-N-isopropylamino group; an N-benzyl-N-isopropylamino group; an N-butyl-N-sec-butylamino group; an N-benzyl-N-sec-butylamino group; an N-butyl-N-tert-butylamino group; an N-benzyl-N-tert-butylamio group; an N-sec-butyl-N-tert-butylamino group; a carboxyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group;
a C₃ to C₇ cycloalkyl group (a cyclopropyl group; a cyclobutyl group; a cyclopentyl group; a cyclohexyl group; a cycloheptyl group; etc.) which may have a substituent (a phenyl group; a methyl group; an ethyl group; a propyl group; a butyl group; a pentyl group; a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.); a C₁ to C₆ alkyl group (a methyl group; an ethyl group; a propyl group; a butyl group; a pentyl group or a hexyl group; an isopropyl group; a sec-butyl group; a tert-butyl group; an isopentyl group; a neopentyl group; a 1-methylpropyl group; an isohexyl group; a 1,1-dimethylbutyl group; a 2,2-dimethylbutyl group; a 3,3-dimethylbutyl group; etc.) having one or more halogen atoms (fluorine; chlorine; bromine; iodine); a carbamoyl group; a methylcarbamoyl group; an ethylcarbamoyl group; a propylcarbamoyl group; an isopropylcarbamoyl group; a butylcarbamoyl group; a sec-butylcarbamoyl group; a tert-butylcarbamoyl group; a dimethylcarbamoyl group; a diethylcarbamoyl group; a dipropylcarbamoyl group; a diisopropylcarbamoyl group; a dibutylcarbamoyl group; a di-sec-butylcarbamoyl group; a di-tert-butylcarbamoyl group; an N-ethyl-N-methylcarbamoyl group; an N-methyl-N-propylcarbamoyl group; an N-isopropyl-N-methylcarbamoyl group; an N-butyl-N-methylcarbamoyl group; an N-sec-butyl-N-methylcarbamoyl group; an N-tert-butyl-N-methylcarbamoyl group; an N-ethyl-N-propylcarbamoyl group; an N-ethyl-N-isopropylcarbamoyl group; an N-butyl-N-ethylcarbamoyl group; an N-sec-butyl-N-ethylcarbamoyl group; an N-tert-butyl-N-ethylcarbamoyl group; an N-isopropyl-N-propylcarbamoyl group; an N-butyl-N-propylcarbamoyl group; an N-sec-butyl-N-propylcarbamoyl group; an N-tert-butyl-N-propylcarbamoyl group; an N-butyl-N-isopropylcarbamoyl group; an N-sec-butyl-N-isopropylcarbamoyl group; an N-tert-butyl-N-isopropylcarbamoyl group; an N-butyl-N-sec-butylcarbamoyl group; an N-butyl-N-tert-butylcarbamoyl group; an N-sec-butyl-N-tert-butylcarbamoyl group; a methoxy group; an ethoxy group; a propoxy group; an isopropoxy group; a butoxy group; a sec-butoxy group; a tert-butoxy group; a pentyloxy group; a hexyloxy group; a methoxycarbonyl group; an ethoxycarbonyl group; a propoxycarbonyl group; an isopropoxycarbonyl group; a butoxycarbonyl group; a sec-butoxycarbonyl group; a tert-butoxycarbonyl group; a pentyloxycarbonyl group; a hexyloxycarbonyl group; a formyl group; an acetyl group; a propionyl group; a butyryl group; an isobutyryl group; a valeryl group; an isovaleryl group; a pivaloyl group; an acryloyl group; a propioyl group; a phenyl group; a naphthyl group;
a 3 to 10-membered monocyclic or fused polycyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; an imidazolidinyl group; an imidazolinyl group; a tetrahydrofuryl group; a pyrazolidinyl group; a pyrazolinyl group; a pyranyl group; a tetrahydropyranyl group; a piperazinyl group; a morpholinyl group; a piperidinyl group; an indolinyl group; an isoindolinyl group; a dihydrobenzofuranyl group; etc.);
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.)},
a 3 to 7-membered monocyclic nonaromatic hetero ring with one or more hetero atoms (an oxiranyl group; an aziridinyl group; an oxetanyl group; a thietanyl group; a thiolanyl group; a thiomorpholinyl group; a pyrrolidinyl group; a pyrrolinyl group; a tetrahydrofuryl group; an imidazolidinyl group; an imidazolinyl group; a pyrazolidinyl group; a pyrazolinyl group; a pyranyl group; a tetrahydropyranyl group; a piperazinyl group; a morpholinyl group; a piperidinyl group; etc.),
a 6 to 10-membered monocyclic or fused polycyclic aryl group (a phenyl group; a naphthyl group; etc.) which may have one or more substituents (a methyl group; a hydroxyl group; a carboxyl group), or
a 5 to 10-membered monocyclic or fused polycyclic heteroaryl group with one or more hetero atoms (a thiazolyl group; a thienyl group; a furyl group; an isothiazolyl group; an oxazolyl group; an isoxazolyl group; a pyrrolyl group; an imidazolyl group; a furazanyl group; a pyrazolyl group; a pyridyl group; a pyrazinyl group; a pyrimidinyl group; a pyridazinyl group; a triazinyl group; a quinolyl group; an isoquinolyl group; an indolyl group; an isoindolyl group; an indolizinyl group; an indazolyl group; a phthalazinyl group; a naphthyridinyl group; a quinoxalinyl group; a quinazolinyl group; a purinyl group; a pteridinyl group; a cinnolinyl group; a chromenyl group; a benzofuranyl group; a benzoxazolyl group; a benzisoxazolyl group; a benzothiazolyl group; a benzisothiazolyl group; a benzimidazolyl group; etc.).

Furthermore, specific substituents of R₁ to R₃ in the formula (I) of the present invention are exemplified below, but the present invention is not limited thereto.

Regarding R₁, for example, a methyl group, an ethyl group, a propyl group, a butyl group, a pentyl group, a hexyl group, an isopropyl group, a sec-butyl group, a tert-butyl group, an isopentyl group, a 3-pentyl group, a neopentyl group, a 1-methylpropyl group, an isohexyl group, a 1,1-dimethylbutyl group, a 2,2-dimethylbutyl group, a 3,3-dimethylbutyl group, a 2-ethylbutyl group, a benzyl group, a phenethyl group, a furylmethyl group, a furylethyl group, a furylpropyl group, a furylbutyl group, a furylpentyl group, a cyclopropyl group, a cyclobutyl group, a cyclopentyl group, a cyclohexyl group, an oxiranyl group, an oxetanyl group, a thietanyl group, a thiolanyl group, a thiomorpholinyl group, a pyrrolidinyl group, a pyrrolinyl group, a tetrahydrofuryl group, an indanyl group, a fluoromethyl group, a fluoroethyl group, a fluoropropyl group, a fluorocyclopropyl group, a fluorocyclopentyl group, a fluorotetrahydrofuryl group, a hydroxymethyl group, a hydroxyethyl group, a hydroxypropyl group, a hydroxycyclopropyl group, a hydroxycyclopentyl group, a hydroxytetrahydrofuryl group, a nitromethyl group, a nitroethyl group, a nitropropyl group, a nitrocyclopropyl group, a nitrocyclopentyl group, a nitrotetrahydrofuryl group, a cyanomethyl group, a cyanoethyl group, a cyanopropyl group, a cyanocyclopropyl group, a cyanocyclopentyl group, a cyanotetrahydrofuryl group, an aminomethyl group, an aminoethyl group, an aminopropyl group, an aminocyclopropyl group, an aminocyclopentyl group, an aminotetrahydrofuryl group, an N-methylaminomethyl group, an N-methylaminoethyl group, an N-methylaminopropyl group, an N-methylaminocyclopropyl group, an N-methylaminocyclopentyl group, an N-methylaminotetrahydrofuryl group, an N-ethylaminomethyl group, an N-ethylaminoethyl group, an N-ethylaminopropyl group, an N-ethylaminocyclopropyl group, an N-ethylaminocyclopentyl group, an N-ethylaminotetrahydrofuryl group, an N-propylaminomethyl group, an N-propylaminoethyl group, an N-propylaminopropyl group, an N-propylaminocyclopropyl group, an N-propylaminocyclopentyl group, an N-propylaminotetrahydrofuryl group, an N-butylaminomethyl group, an N-butylaminoethyl group, an N-butylaminopropyl group, an N-butylaminocyclopropyl group, an N-butylaminocyclopentyl group, an N-butylaminotetrahydrofuryl group, an N-benzylaminomethyl group, an N-benzylaminoethyl group, an N-benzylaminopropyl group, an N-benzylaminocyclopropyl group, an N-benzylaminocyclopentyl group, an N-benzylaminotetrahydrofuryl group, an N,N-dimethylaminomethyl group, an N,N-dimethylaminoethyl group, an N,N-dimethylaminopropyl group, an N,N-dimethylaminocyclopropyl group, an N,N-dimethylaminocyclopentyl group, an N,N-dimethylaminotetrahydrofuryl group, an N,N-diethylaminomethyl group, an N,N-diethylaminoethyl group, an N,N-diethylaminopropyl group, an N,N-diethylaminocyclopropyl group, an N,N-diethylaminocyclopentyl group, an N,N-diethylaminotetrahydrofuryl group, an N,N-dipropylaminomethyl group, an N,N-dipropylaminoethyl group, an N,N-dipropylaminopropyl group, an N,N-dipropylaminocyclopropyl group, an N,N-dipropylaminocyclopentyl group, an N,N-dipropylaminotetrahydrofuryl group, an N,N-dibutylaminomethyl group, an N,N-dibutylaminoethyl group, an N,N-dibutylaminopropyl group, an N,N-dibutylaminocyclopropyl group, an N,N-dibutylaminocyclopentyl group, an N,N-dibutylaminotetrahydrofuryl group, an N,N-dibenzylaminomethyl group, an N,N-dibenzylaminoethyl group, an N,N-dibenzylaminopropyl group, an N,N-dibenzylaminocyclopropyl group, an N,N-dibenzylaminocyclopentyl group, an N,N-dibenzylaminotetrahydrofuryl group, an N-methyl-N-ethylaminomethyl group, an N-methyl-N-ethylaminoethyl group, an N-methyl-N-ethylaminopropyl group, an N-methyl-N-ethylaminocyclopropyl group, an N-methyl-N-ethylaminocyclopentyl group, an N-methyl-N-ethylaminotetrahydrofuryl group, an N-methyl-N-propylaminomethyl group, an N-methyl-N-propylaminoethyl group, an N-methyl-N-propylaminopropyl group, an N-methyl-N-propylaminocyclopropyl group, an N-methyl-N-propylaminocyclopentyl group, an N-methyl-N-propylaminotetrahydrofuryl group, an N-methyl-N-butylaminomethyl group, an N-methyl-N-butylaminoethyl group, an N-methyl-N-butylaminopropyl group, an N-methyl-N-butylaminocyclopropyl group, an N-methyl-N-butylaminocyclopentyl group, an N-methyl-N-butylaminotetrahydrofuryl group, an N-methyl-N-benzylaminomethyl group, an N-methyl-N-benzylaminoethyl group, an N-methyl-N-benzylaminopropyl group, an N-methyl-N-benzylaminocyclopropyl group, an N-methyl-N-benzylaminocyclopentyl group, an N-methyl-N-benzylaminotetrahydrofuryl group, an N-ethyl-N-propylaminomethyl group, an N-ethyl-N-propylaminoethyl group, an N-ethyl-N-propylaminopropyl group, an N-ethyl-N-propylaminocyclopropyl group, an N-ethyl-N-propylaminocyclopentyl group, an N-ethyl-N-propylaminotetrahydrofuryl group, an N-ethyl-N-butylaminomethyl group, an N-ethyl-N-butylaminoethyl group, an N-ethyl-N-butylaminopropyl group, an N-ethyl-N-butylaminocyclopropyl group, an N-ethyl-N-butylaminocyclopentyl group, an N-ethyl-N-butylaminotetrahydrofuryl group, an N-ethyl-N-benzylaminomethyl group, an N-ethyl-N-benzylaminoethyl group, an N-ethyl-N-benzylaminopropyl group, an N-ethyl-N-benzylaminocyclopropyl group, an N-ethyl-N-benzylaminocyclopentyl group, an N-ethyl-N-benzylaminotetrahydrofuryl group, an N-propyl-N-butylaminomethyl group, an N-propyl-N-butylaminoethyl group, an N-propyl-N-butylaminopropyl group, an N-propyl-N-butylaminocyclopropyl group, an N-propyl-N-butylaminocyclopentyl group, an N-propyl-N-butylaminotetrahydrofuryl group, an N-propyl-N-benzylaminomethyl group, an N-propyl-N-benzylaminoethyl group, an N-propyl-N-benzylaminopropyl group, an N-propyl-N-benzylaminocyclopropyl group, an N-propyl-N-benzylaminocyclopentyl group, an N-propyl-N-benzylaminotetrahydrofuryl group, an N-butyl-N-benzylaminomethyl group, an N-butyl-N-benzylaminoethyl group, an N-butyl-N-benzylaminopropyl group, an N-butyl-N-benzylaminocyclopropyl group, an N-butyl-N-benzylaminocyclopentyl group, an N-butyl-N-benzylaminotetrahydrofuryl group, an acetylmethyl group, an acetylethyl group, an acetylpropyl group, an acetylcyclopropyl group, an acetylcyclopentyl group, a methylcyclopropyl group, a methylcyclopentyl group, a methyltetrahydrofuryl group, an ethylcyclopropyl group, an ethylcyclopentyl group, an ethyltetrahydrofuryl group, a cyclopropylmethyl group, a phenylcyclopropylmethyl group, a phenylcyclopropylethyl group, a phenylcyclopropylpropyl group, a phenylcyclopropylcyclopropyl group, a phenylcyclopropylcyclopentyl group, a phenylcyclopropyltetrahydrofuryl group, a phenylcyclopentylmethyl group, a phenylcyclopentylethyl group, a phenylcyclopentylpropyl group, a methylcyclopropylmethyl group, a methylcyclopropylethyl group, a methylcyclopropylpropyl group, a methylcyclopropyltetrahydrofuryl group, a methylcyclopentylmethyl group, a methylcyclopentylethyl group, a methylcyclopentylpropyl group, a methylcyclopentylcyclopropyl group, a methylcyclopentylcyclopentyl group, a methylcyclopentyltetrahydrofuryl group, a fluoromethylcyclopropyl group, a fluoromethylcyclopentyl group, a fluoromethyltetrahydrofuryl group, a carbamoylmethyl group, a carbamoylethyl group, a carbamoylpropyl group, a methylcarbamoylmethyl group, an ethylcarbamoylmethyl group, a propylcarbamoylmethyl group, an isopropylcarbamoylmethyl group, a butylcarbamoylmethyl group, a sec-butylcarbamoylmethyl group, a tert-butylcarbamoylmethyl group, a dimethylcarbamoylmethyl group, a diethylcarbamoylmethyl group, a dipropylcarbamoylmethyl group, a diisopropylcarbamoylmethyl group, a dibutylcarbamoylmethyl group, a di-sec-butylcarbamoylmethyl group, a di-tert-butylcarbamoylmethyl group, an N-ethyl-N-methylcarbamoylmethyl group, an N-methyl-N-propylcarbamoylmethyl group, an N-isopropyl-N-methylcarbamoylmethyl group, an N-butyl-N-methylcarbamoylmethyl group, an N-sec-butyl-N-methylcarbamoylmethyl group, an N-tert-butyl-N-methylcarbamoylmethyl group, an N-ethyl-N-propylcarbamoylmethyl group, an N-ethyl-N-isopropylcarbamoylmethyl group, an N-butyl-N-ethylcarbamoylmethyl group, an N-sec-butyl-N-ethylcarbamoylmethyl group, an N-tert-butyl-N-ethylcarbamoylmethyl group, an N-isopropyl-N-propylcarbamoylmethyl group, an N-butyl-N-propylcarbamoylmethyl group, an N-sec-butyl-N-tert-butylcarbamoylmethyl group, an N-sec-butyl-N-propylcarbamoylmethyl group, an N-tert-butyl-N-propylcarbamoylmethyl group, an N-butyl-N-isopropylcarbamoylmethyl group, an N-sec-butyl-N-isopropylcarbamoylmethyl group, an N-tert-butyl-N-isopropylcarbamoylmethyl group, an N-butyl-N-sec-butylcarbamoylmethyl group, an N-butyl-N-tert-butylcarbamoylmethyl group, a methylcarbamoylethyl group, a dimethylcarbamoylethyl group, a diethylcarbamoylethyl group, an ethylcarbamoylethyl group, a methylcarbamoylpropyl group, an ethylcarbamoylpropyl group, a dimethylcarbamoylpropyl group, a diethylcarbamoylpropyl group, a methylcarbamoylbutyl group, an ethylcarbamoylbutyl group, a dimethylcarbamoylbutyl group, a diethylcarbamoylbutyl group, an ethylcarbamoylpentyl group, a propylcarbamoylpentyl group, a dimethylcarbamoylpentyl group, a diethylcarbamoylpentyl group, a carbamoylcyclopropyl group, a carbamoylcyclopentyl group, a carbamoyltetrahydrofuryl group, a methoxymethyl group, a methoxyethyl group, a methoxypropyl group, a methoxycyclopropyl group, a methoxycyclopentyl group, a methoxytetrahydrofuryl group, an ethoxymethyl group, an ethoxyethyl group, an ethoxypropyl group, an ethoxycyclopropyl group, an ethoxycyclopentyl group, an ethoxytetrahydrofuryl group, a methoxycarbonylmethyl group, a methoxycarbonylethyl group, a methoxycarbonylpropyl group, a methoxycarbonylcyclopropyl group, a methoxycarbonylcyclopentyl group, a methoxycarbonyltetrahydrofuryl group, a pyridylmethyl group, a pyridylethyl group, a pyridylpropyl group, a pyridylcyclopropyl group, a pyridylcyclopentyl group, a pyridyltetrahydrofuryl group, a phenylpropyl group, a phenylcyclopropyl group, a phenylcyclopentyl group, a phenyltetrahydrofuryl group, a thiazolylmethyl group, a thiazolylethyl group, a thiazolylpropyl group, a thiazolylcyclopropyl group, a thiazolylcyclopentyl group, a furylmethyl group, a furylethyl group, a furylpropyl group, a furylcyclopropyl group, a thienylmethyl group, a thienylethyl group, a thienylpropyl group, a thienylcyclopropyl group, a thienylcyclopentyl group, a naphthylmethyl group, a naphthylethyl group, a naphthylpropyl group, a naphthylcyclopropyl group, and a naphthylcyclopentyl group are exemplified.

Regarding R₂, for example, a methyl group, an ethyl group, a propyl group, a butyl group, a trifluoromethyl group, a difluoromethyl group, a fluoromethyl group, a cyclopropyl group, a cyclobutyl group, a cyclopentyl group, a cyclohexyl group, a fluorocyclopropyl group, a fluorocyclopentyl group, a hydroxycyclopropyl group, a hydroxycyclopentyl group, a nitrocyclopropyl group, a nitrocyclopentyl group, a cyanocyclopropyl group, a cyanocyclopentyl group, an aminocyclopropyl group, an aminocyclopentyl group, an N-methylaminocyclopropyl group, an N-methylaminocyclopentyl group, an N-ethylaminocyclopropyl group, an N-ethylaminocyclopentyl group, an N-butylaminocyclopropyl group, an N-butylaminocyclopentyl group, an N-benzylaminocyclopropyl group, an N-benzylaminocyclopentyl group, an N,N-dimethylaminocyclopropyl group, an N,N-dimethylaminocyclopentyl group, an N,N-diethylaminocyclopropyl group, an N,N-diethylaminocyclopentyl group, an N,N-dipropylaminocyclopropyl group, an N,N-dipropylaminocyclopentyl group, an N,N-dibutylaminocyclopropyl group, an N,N-dibutylaminocyclopentyl group, an N,N-dibenzylaminocyclopropyl group, an N,N-dibenzylaminocyclopentyl group, an N-methyl-N-ethylaminocyclopropyl group, an N-methyl-N-ethylaminocyclopentyl group, an N-methyl-N-propylaminocyclopropyl group, an N-methyl-N-propylaminocyclopentyl group, an N-methyl-N-butylaminocyclopropyl group, an N-methyl-N-butylaminocyclopentyl group, an N-methyl-N-benzylaminocyclopropyl group, an N-methyl-N-benzylaminocyclopentyl group, an N-ethyl-N-propylaminocyclopropyl group, an N-ethyl-N-propylaminocyclopentyl group, an N-ethyl-N-butylaminocyclopropyl group, an N-ethyl-N-butylaminocyclopentyl group, an N-ethyl-N-benzylaminocyclopropyl group, an N-ethyl-N-benzylaminocyclopentyl group, an N-propyl-N-butylaminocyclopropyl group, an N-propyl-N-butylaminocyclopentyl group, an N-propyl-N-benzylaminocyclopropyl group, an N-propyl-N-benzylaminocyclopentyl group, an N-butyl-N-benzylaminocyclopropyl group, and an N-butyl-N-benzylaminocyclopentyl group are exemplified.

Regarding R₃, for example, a hydrogen atom, a methyl group, an ethyl group, a hydroxycarbonylmethyl group, a butyl group, a phenyl group, a tolyl group, a xylyl group, a hydroxyphenyl group, a dihydroxyphenyl group, a trihydroxyphenyl group, a hydroxycarbonylphenyl group, a dihydroxycarbonylphenyl group, a trihydroxycarbonylphenyl group, a benzyl group, a phenethyl group, a pyridylmethyl group, a pyridylethyl group, a fluoromethyl group, a fluoroethyl group, a fluoropropyl group, a hydroxymethyl group, a hydroxyethyl group, a hydroxypropyl group, a nitromethyl group, a nitroethyl group, a nitropropyl group, a cyanomethyl group, a cyanoethyl group, a cyanopropyl group, an aminomethyl group, an aminoethyl group, an aminopropyl group, an aminocyclopropyl group, an aminocyclopentyl group, an aminotetrahydrofuryl group, an N-methylaminomethyl group, an N-methylaminoethyl group, an N-methylaminopropyl group, an N-methylaminocyclopropyl group, an N-methylaminocyclopentyl group, an N-methylaminotetrahydrofuryl group, an N-ethylaminomethyl group, an N-ethylaminoethyl group, an N-ethylaminopropyl group, an N-ethylaminocyclopropyl group, an N-ethylaminocyclopentyl group, an N-ethylaminotetrahydrofuryl group, an N-propylaminomethyl group, an N-propylaminoethyl group, an N-propylaminopropyl group, an N-propylaminocyclopropyl group, an N-propylaminocyclopentyl group, an N-propylaminotetrahydrofuryl group, an N-butylaminomethyl group, an N-butylaminoethyl group, an N-butylaminopropyl group, an N-butylaminocyclopropyl group, an N-butylaminocyclopentyl group, an N-butylaminotetrahydrofuryl group, an N-benzylaminomethyl group, an N-benzylaminoethyl group, an N-benzylaminopropyl group, an N-benzylaminocyclopropyl group, an N-benzylaminocyclopentyl group, an N-benzylaminotetrahydrofuryl group, an N,N-dimethylaminomethyl group, an N,N-dimethylaminoethyl group, an N,N-dimethylaminopropyl group, an N,N-dimethylaminocyclopropyl group, an N,N-dimethylaminocyclopentyl group, an N,N-dimethylaminotetrahydrofuryl group, an N,N-diethylaminomethyl group, an N,N-diethylaminoethyl group, an N,N-diethylaminopropyl group, an N,N-diethylaminocyclopropyl group, an N,N-diethylaminocyclopentyl group, an N,N-diethylaminotetrahydrofuryl group, an N,N-dipropylaminomethyl group, an N,N-dipropylaminoethyl group, an N,N-dipropylaminopropyl group, an N,N-dipropylaminocyclopropyl group, an N,N-dipropylaminocyclopentyl group, an N,N-dipropylaminotetrahydrofuryl group, an N,N-dibutylaminomethyl group, an N,N-dibutylaminoethyl group, an N,N-dibutylaminopropyl group, an N,N-dibutylaminocyclopropyl group, an N,N-dibutylaminocyclopentyl group, an N,N-dibutylaminotetrahydrofuryl group, an N,N-dibenzylaminomethyl group, an N,N-dibenzylaminoethyl group, an N,N-dibenzylaminopropyl group, an N,N-dibenzylaminocyclopropyl group, an N,N-dibenzylaminocyclopentyl group, an N,N-dibenzylaminotetrahydrofuryl group, an N-methyl-N-ethylaminomethyl group, an N-methyl-N-ethylaminoethyl group, an N-methyl-N-ethylaminopropyl group, an N-methyl-N-ethylaminocyclopropyl group, an N-methyl-N-ethylaminocyclopentyl group, an N-methyl-N-ethylaminotetrahydrofuryl group, an N-methyl-N-propylaminomethyl group, an N-methyl-N-propylaminoethyl group, an N-methyl-N-propylaminopropyl group, an N-methyl-N-propylaminocyclopropyl group, an N-methyl-N-propylaminocyclopentyl group, an N-methyl-N-propylaminotetrahydrofuryl group, an N-methyl-N-butylaminomethyl group, an N-methyl-N-butylaminoethyl group, an N-methyl-N-butylaminopropyl group, an N-methyl-N-butylaminocyclopropyl group, an N-methyl-N-butylaminocyclopentyl group, an N-methyl-N-butylaminotetrahydrofuryl group, an N-methyl-N-benzylaminomethyl group, an N-methyl-N-benzylaminoethyl group, an N-methyl-N-benzylaminopropyl group, an N-methyl-N-benzylaminocyclopropyl group, an N-methyl-N-benzylaminocyclopentyl group, an N-methyl-N-benzylaminotetrahydrofuryl group, an N-ethyl-N-propylaminomethyl group, an N-ethyl-N-propylaminoethyl group, an N-ethyl-N-propylaminopropyl group, an N-ethyl-N-propylaminocyclopropyl group, an N-ethyl-N-propylaminocyclopentyl group, an N-ethyl-N-propylaminotetrahydrofuryl group, an N-ethyl-N-butylaminomethyl group, an N-ethyl-N-butylaminoethyl group, an N-ethyl-N-butylaminopropyl group, an N-ethyl-N-butylaminocyclopropyl group, an N-ethyl-N-butylaminocyclopentyl group, an N-ethyl-N-butylaminotetrahydrofuryl group, an N-ethyl-N-benzylaminomethyl group, an N-ethyl-N-benzylaminoethyl group, an N-ethyl-N-benzylaminopropyl group, an N-ethyl-N-benzylaminocyclopropyl group, an N-ethyl-N-benzylaminocyclopentyl group, an N-ethyl-N-benzylaminotetrahydrofuryl group, an N-propyl-N-butylaminomethyl group, an N-propyl-N-butylaminoethyl group, an N-propyl-N-butylaminopropyl group, an N-propyl-N-butylaminocyclopropyl group, an N-propyl-N-butylaminocyclopentyl group, an N-propyl-N-butylaminotetrahydrofuryl group, an N-propyl-N-benzylaminomethyl group, an N-propyl-N-benzylaminoethyl group, an N-propyl-N-benzylaminopropyl group, an N-propyl-N-benzylaminocyclopropyl group, an N-propyl-N-benzylaminocyclopentyl group, an N-propyl-N-benzylaminotetrahydrofuryl group, an N-butyl-N-benzylaminomethyl group, an N-butyl-N-benzylaminoethyl group, an N-butyl-N-benzylaminopropyl group, an N-butyl-N-benzylaminocyclopropyl group, an N-butyl-N-benzylaminocyclopentyl group, an N-butyl-N-benzylaminotetrahydrofuryl group, an acetylmethyl group, an acetylethyl group, an acetylpropyl group, a phenylcyclopropylmethyl group, a phenylcyclopropylethyl group, a phenylcyclopropylpropyl group, a phenylcyclopentylmethyl group, a phenylcyclopentylethyl group, a phenylcyclopentylpropyl group, a methylcyclopropylmethyl group, a methylcyclopropylethyl group, a methylcyclopropylpropyl group, a methylcyclopropyltetrahydrofuryl group, a methylcyclopentylmethyl group, a methylcyclopentylethyl group, a methylcyclopentylpropyl group, a carbamoylmethyl group, a carbamoylethyl group, a carbamoylpropyl group, a methylcarbamoylmethyl group, an ethylcarbamoylmethyl group, a propylcarbamoylmethyl group, an isopropylcarbamoylmethyl group, a butylcarbamoylmethyl group, a sec-butylcarbamoylmethyl group, a tert-butylcarbamoylmethyl group, a dimethylcarbamoylmethyl group, a diethylcarbamoylmethyl group, a dipropylcarbamoylmethyl group, a diisopropylcarbamoylmethyl group, a dibutylcarbamoylmethyl group, a di-sec-butylcarbamoylmethyl group, a di-tert-butylcarbamoylmethyl group, an N-ethyl-N-methylcarbamoylmethyl group, an N-methyl-N-propylcarbamoylmethyl group, an N-isopropyl-N-methylcarbamoylmethyl group, an N-butyl-N-methylcarbamoylmethyl group, an N-sec-butyl-N-methylcarbamoylmethyl group, an N-tert-butyl-N-methylcarbamoylmethyl group, an N-ethyl-N-propylcarbamoylmethyl group, an N-ethyl-N-isopropylcarbamoylmethyl group, an N-butyl-N-ethylcarbamoylmethyl group, an N-sec-butyl-N-ethylcarbamoylmethyl group, an N-tert-butyl-N-ethylcarbamoylmethyl group, an N-isopropyl-N-propylcarbamoylmethyl group, an N-butyl-N-propylcarbamoylmethyl group, an N-sec-butyl-N-tert-butylcarbamoylmethyl group, an N-sec-butyl-N-propylcarbamoylmethyl group, an N-tert-butyl-N-propylcarbamoylmethyl group, an N-butyl-N-isopropylcarbamoylmethyl group, an N-sec-butyl-N-isopropylcarbamoylmethyl group, an N-tert-butyl-N-isopropylcarbamoylmethyl group, an N-butyl-N-sec-butylcarbamoylmethyl group, an N-butyl-N-tert-butylcarbamoylmethyl group, a methylcarbamoylethyl group, a dimethylcarbamoylethyl group, a diethylcarbamoylethyl group, an ethylcarbamoylethyl group, a methylcarbamoylpropyl group, an ethylcarbamoylpropyl group, a dimethylcarbamoylpropyl group, a diethylcarbamoylpropyl group, a methylcarbamoylbutyl group, an ethylcarbamoylbutyl group, a dimethylcarbamoylbutyl group, a diethylcarbamoylbutyl group, an ethylcarbamoylpentyl group, a propylcarbamoylpentyl group, a dimethylcarbamoylpentyl group, a diethylcarbamoylpentyl group, a methoxymethyl group, a methoxyethyl group, a methoxypropyl group, an ethoxymethyl group, an ethoxyethyl group, an ethoxypropyl group, a methoxycarbonylmethyl group, a methoxycarbonylethyl group, a methoxycarbonylpropyl group, a pyridylpropyl group, a phenylpropyl group, a thiazolylmethyl group, a thiazolylethyl group, a thiazolylpropyl group, a furylmethyl group, a furylethyl group, a furylpropyl group, a thienylmethyl group, a thienylethyl group, a thienylpropyl group, a naphthylmethyl group, a naphthylethyl group, a naphthylpropyl group, a cyclopropyl group, a cyclopentyl group, a tetrahydrofuryl group, a methyltetrahydrofuryl group, a hydroxytetrahydrofuryl group, a hydroxycarbonyltetrahydrofuryl group, a pyridyl group, a furyl group, a thiazolyl group, a thienyl group, an isothiazolyl group, an oxazolyl group, an isoxazolyl group, a pyrrolyl group, an imidazolyl group, a furazanyl group, a pyrazolyl group, a pyridyl group, a pyrazinyl group, a pyrimidinyl group, a pyridazinyl group, a triazinyl group, a quinolyl group, an isoquinolyl group, an indolyl group, an isoindolyl group, an indolizinyl group, an indazolyl group, a phthalazinyl group, a naphthyridinyl group, a quinoxalinyl group, a quinazolinyl group, a purinyl group, a pteridinyl group, a cinnolinyl group, a chromenyl group, a benzofuranyl group, a benzoxazolyl group, a benzisoxazolyl group, a benzothiazolyl group, a benzisothiazolyl group, a benzimidazolyl group, a 2-piperidinylmethyl group, a 3-piperidinylmethyl group, a 4-piperidinylmethyl group, a 2-piperidinylethyl group, a 3-piperidinylethyl group, a 4-piperidinylethyl group, a (1-methylpiperidin-2-yl)methyl group, a (1-methylpiperidin-3-yl)methyl group, a (1-methylpiperidin-4-yl)methyl group, a (1-methylpiperidin-2-yl)ethyl group, a (1-methylpiperidin-3-yl)ethyl group, a (1-methylpiperidin-4-yl)ethyl group, a methyloxiranyl group, a methylaziridinyl group, a methyloxetanyl group, a methylthietanyl group, a methylthiolanyl group, a methylthiomorpholinyl group, a methyltetrahydrofuryl group, a methylpyrrolidinyl group, a methylpyrrolinyl group, a methylimidazolidinyl group, a methylimidazolinyl group, a methylpyrazolidinyl group, a methylpyrazolinyl group, a methylpyranyl group, a methyltetrahydropyranyl group, a methylpiperazinyl group, a methylmorpholinyl group, a methylpiperidinyl group, a methylindolinyl group, a methylisoindolinyl group, a methyldihydrobenzofuranyl group, a methyltetrahydroquinolyl group, a methyltetrahydroisoquinolyl group, a hydroxyoxiranyl group, a hydroxyaziridinyl group, a hydroxyoxetanyl group, a hydroxythietanyl group, a hydroxythiolanyl group, a hydroxythiomorpholinyl group, a hydroxytetrahydrofuryl group, a hydroxypyrrolidinyl group, a hydroxypyrrolinyl group, a hydroxyimidazolidinyl group, a hydroxyimidazolinyl group, a hydroxypyrazolidinyl group, a hydroxypyrazolinyl group, a hydroxypyranyl group, a hydroxytetrahydropyranyl group, a hydroxypiperazinyl group, a hydroxymorpholinyl group, a hydroxypiperidinyl group, a hydroxyindolinyl group, a hydroxyisoindolinyl group, a hydroxydihydrobenzofuranyl group, a hydroxytetrahydroquinolyl group, a hydroxytetrahydroisoquinolyl group, a hydroxycarbonyloxiranyl group, a hydroxycarbonylaziridinyl group, a hydroxycarbonyloxetanyl group, a hydroxycarbonylthietanyl group, a hydroxycarbonylthiolanyl group, a hydroxycarbonylthiomorpholinyl group, a hydroxycarbonyltetrahydrofuryl group, a hydroxycarbonylpyrrolidinyl group, a hydroxycarbonylpyrrolinyl group, a hydroxycarbonylimidazolidinyl group, a hydroxycarbonylimidazolinyl group, a hydroxycarbonylpyrazolidinyl group, a hydroxycarbonylpyrazolinyl group, a hydroxycarbonylpyranyl group, a hydroxycarbonyltetrahydropyranyl group, a hydroxycarbonylpiperazinyl group, a hydroxycarbonylmorpholinyl group, a hydroxycarbonylpiperidinyl group, a hydroxycarbonylindolinyl group, a hydroxycarbonylisoindolinyl group, a hydroxycarbonyldihydrobenzofuranyl group, a hydroxycarbonyltetrahydroquinolyl group, a hydroxycarbonyltetrahydroisoquinolyl group, etc. are exemplified.

A C₁ to C₈ alkyl group of the present invention represents a chain group composed of 1 to 8 carbons only, and, for example,
a methyl group, an ethyl group, a propyl group, a butyl group, an isopropyl group, a sec-butyl group, a tert-butyl group, a pentyl group, an isopentyl group, a 3-pentyl group, a neopentyl group, a 1-methylpropyl group, a hexyl group, an isohexyl group, a heptyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group, a 2-ethylbutyl group, a 1,1-diethylbutyl group, a 1,2-diethylbutyl group, a 2,2-diethylbutyl group, a 1,1,2-trimethylbutyl group, a 1,1,3-trimethylbutyl group, a 1,2,2-trimethylbutyl group, a 1,2,3-trimethylbutyl group, a 1,3,3-trimethylbutyl group, a 2,2,3-trimethylbutyl group, a 2,3,3-trimethylbutyl group, a 1-methylpentyl group, a 2-methylpentyl group, a 3-methylpentyl group, a 4-methylpentyl group, a 1,1-dimethylpentyl group, a 1,2-dimethylpentyl group, a 1,3-dimethylpentyl group, a 1,4-dimethylpentyl group, a 2,2-dimethylpentyl group, a 2,3-dimethylpentyl group, a 2,4-dimethylpentyl group, a 3,3-dimethylpentyl group, a 3,4-dimethylpentyl group, a 4,4-dimethylpentyl group, a 1,1,2-trimethylpentyl group, a 1,1,3-trimethylpentyl group, a 1,1,4-trimethylpentyl group, a 1,2,2-trimethylpentyl group, a 1,2,3-trimethylpentyl group, a 1,2,4-trimethylpentyl group, a 1,3,3-trimethylpentyl group, a 1,3,4-trimethylpentyl group, a 1,4,4-trimethylpentyl group, a 2,2,3-trimethylpentyl group, a 2,2,4-trimethylpentyl group, a 2,3,4-trimethylpentyl group, a 2,4,4-trimethylpentyl group, a 3,3,4-trimethylpentyl group, a 3,4,4-trimethylpentyl group, a 1-ethylpentyl group, a 2-ethylpentyl group, a 3-ethylpentyl group, a 4-ethylpentyl group, and an octyl group, etc. are exemplified.

A C₁ to C₆ alkyl group of the present invention represents a chain group composed of 1 to 6 carbons only, and, for example,
a methyl group, an ethyl group, a propyl group, a butyl group, an isopropyl group, a sec-butyl group, a tert-butyl group, a pentyl group, a hexyl group, an isopentyl group, a 3-pentyl group, a neopentyl group, a 1-methylpropyl group, a 1-methylpentyl group, a 2-methylpentyl group, a 3-methylpentyl group, an isohexyl group, a 2-ethylbutyl group, a 1,1-dimethylbutyl group, a 1,2-dime-thylbutyl group, a 1,3-dimethylbutyl group, a 2,2-dimethylbutyl group, a 2,3-dimethylbutyl group, a 3,3-dimethylbutyl group, a 1-ethylbutyl group, a 2-ethylbutyl group, a 1,1-diethylbutyl group, a 1,2-diethylbutyl group, a 2,2-diethylbutyl group, etc. are exemplified.

A C₁ to C₅ alkyl group of the present invention represents a chain group composed of 1 to 5 carbons only, and, for example,
a methyl group, an ethyl group, a propyl group, a butyl group, an isopropyl group, a sec-butyl group, a tert-butyl group, an isopentyl group, a 3-pentyl group, a neopentyl group, an 1-methylbutyl group, an 2-methylbutyl group, or a pentyl group, etc. are exemplified.

A C₁ to C₄ alkyl group of the present invention represents a chain group composed of 1 to 4 carbons only, and for example,
a methyl group, an ethyl group, a propyl group, a butyl group, an isopropyl group, a sec-butyl group, a tert-butyl group, etc. are exemplified.

A C₁ to C₃ alkyl group of the present invention represents a chain group composed of 1 to 3 carbons only, and, for example,
a methyl group, an ethyl group, a propyl group, an isopropyl group, etc. are exemplified.

A C₁ to C₂ alkyl group of the present invention represents a chain group composed of 1 or 2 carbons only, which indicates a methyl group and an ethyl group.

A C₃ to C₇ cycloalkyl group of the present invention represents a cyclic group composed of 3 to 7 carbons only, which indicates a cyclopropyl group, a cyclobutyl group, a cyclopentyl group, a cyclohexyl group, a cycloheptyl group, etc.

A C₄ to C₆ cycloalkyl group of the present invention represents a cyclic group composed of 4 to 6 carbons only, which indicates a cyclobutyl group, a cyclopentyl group, a cyclohexyl group.

A halogen atom of the present invention indicates fluorine, chlorine, bromine, and iodine.

A C₁ to C₆ haloalkyl group of the present invention indicates a C₁ to C₆ alkyl group having one or more halogen atoms which are selected from the group consisting of fluorine, chlorine, bromine, and iodine, and, for example,
a fluoromethyl group, a chloromethyl group, a bromomethyl group, an iodomethyl group, a difluoromethyl group, a dichloromethyl group, a dibromomethyl group, a diiodomethyl group, a trifluoromethyl group, a trichloromethyl group, a tribromomethyl group, a triiodomethyl group, a fluoroethyl group, a chloroethyl group, a bromoethyl group, an iodoethyl group, a fluoropropyl group, a chloropropyl group, a bromopropyl group, an iodopropyl group, a fluorobutyl group, a chlorobutyl group, a bromobutyl group, an iodobutyl group, a fluoropentyl group, a chloropentyl group, a bromopentyl group, an iodopentyl group, a fluorohexyl group, a chlorohexyl group, a bromohexyl group, an iodohexyl group, etc. are exemplified.

A C₁ to C₄ haloalkyl group of the present invention indicates a C₁ to C₄ alkyl group having one or more halogen atoms which are selected from the group consisting of fluorine, chlorine, bromine, and iodine, and, for example,
a fluoromethyl group, a chloromethyl group, a bromomethyl group, an iodomethyl group, a difluoromethyl group, a dichloromethyl group, a dibromomethyl group, a diiodomethyl group, a trifluoromethyl group, a trichloromethyl group, a tribromomethyl group, a triiodomethyl group, a fluoroethyl group, a chloroethyl group, a bromoethyl group, an iodoethyl group, a fluoropropyl group, a chloropropyl group, a bromopropyl group, an iodopropyl group, a fluorobutyl group, a chlorobutyl group, a bromobutyl group, an iodobutyl group, etc. are exemplified.

An alkoxy group of the present invention indicates an atom group represented by R-O (R represents an alkyl group), thereby a C₁ to C₆ alkoxy group representing an alkoxy group composed of 1 to 6 carbons, and, for example,
a methoxy group, an ethoxy group, a propoxy group, an isopropoxy group, a butoxy group, a sec-butoxy group, a tert-butoxy group, a pentyloxy group, a hexyloxy group, etc. are exemplified.

An acyl group of the present invention indicates an atom group represented by the form (R-CO-) of carboxylic acid (R-CO-OH: R represents an alkyl group) with the hydroxyl group (OH) eliminated, thereby a C₁ to C₆ acyl group representing an acyl group composed of 1 to 6 carbons, and, for example,
a formyl group; an acetyl group; a propionyl group; a butyryl group; an isobutyryl group; a valeryl group; an isovaleryl group; a pivaloyl group; an acryloyl group; a propioyl group are exemplified.

An alkoxycarbonyl group of the present invention indicates an atom group represented by R-O-CO- (R represents an alkyl group), thereby a C₁ to C₆ alkoxycarbonyl group representing an alkoxycarbonyl group composed of 1 to 6 carbons for its alkyl group part, and, for example,
a methoxycarbonyl group, an ethoxycarbonyl group, a propoxycarbonyl group, an isopropoxycarbonyl group, a butoxycarbonyl group, a sec-butoxycarbonyl group, a tert-butoxycarbonyl group, a pentyloxycarbonyl group, a hexyloxycarbonyl group, etc. are exemplified.

A hetero atom of the present invention represents an atom other than hydrogen and carbon, and, for example,
an oxygen atom, a nitrogen atom, and a sulfur atom, etc. are exemplified.

A carbamoyl group which may have an alkyl group as a substituent on the nitrogen atom of the present invention indicates an atom group represented by (R)(R')-N-CO- (R and R' each independently represent a hydrogen atom or an alkyl group), thereby a carbamoyl group which may have a C₁ to C₄ alkyl group as a substituent representing a carbamoyl group which may have 1 to 4 carbons for the alkyl group part, and, for example,
a carbamoyl group, a methylcarbamoyl group, an ethylcarbamoyl group, a propylcarbamoyl group, an isopropylcarbamoyl group, a butylcarbamoyl group, a sec-butylcarbamoyl group, a tert-butylcarbamoyl group, a dimethylcarbamoyl group, a diethylcarbamoyl group, a dipropylcarbamoyl group, a diisopropylcarbamoyl group, a dibutylcarbamoyl group, a di-sec-butylcarbamoyl group, a di-tert-butylcarbamoyl group, an N-ethyl-N-methylcarbamoyl group, an N-methyl-N-propylcarbamoyl group, an N-isopropyl-N-methylcarbamoyl group, an N-butyl-N-methylcarbamoyl group, an N-sec-butyl-N-methylcarbamoyl group, an N-tert-butyl-N-methylcarbamoyl group, an N-ethyl-N-propylcarbamoyl group, an N-ethyl-N-isopropylcarbamoyl group, an N-butyl-N-ethylcarbamoyl group, an N-sec-butyl-N-ethylcarbamoyl group, an N-tert-butyl-N-ethylcarbamoyl group, an N-isopropyl-N-propylcarbamoyl group, an N-butyl-N-propylcarbamoyl group, an N-sec-butyl-N-propylcarbamoyl group, an N-tert-butyl-N-propylcarbamoyl group, an N-butyl-N-isopropylcarbamoyl group, an N-sec-butyl-N-isopropylcarbamoyl group, an N-tert-butyl-N-isopropylcarbamoyl group, an N-butyl-N-sec-butylcarbamoyl group, an N-butyl-N-tert-butylcarbamoyl group, an N-sec-butyl-N-tert-butylcarbamoyl group, etc. are exemplified.

An amino group which may have an alkyl group or a benzyl group as a substituent on the nitrogen atom of the present invention indicates an atom group represented by (R)(R')-N- (R and R' each independently represent a hydrogen atom; an alkyl group; or a benzyl group), thereby am amino group which may have a C₁ to C₄ alkyl group or a benzyl group as a substituent representing an amino group which may have 1 to 4 carbons or a benzyl group for the alkyl group part, and, for example,
an amino group, a methylamino group, an ethylamino group, a propylamino group, an isopropylamino group, a butylamino group, a sec-butylamino group, a tert-butylamino group, a benzylamino group, a dimethylamino group, a diethylamino group, a dipropylamino group, a diisopropylamino group, a dibutylamino group, a di-sec-butylamino group, a di-tert-butylamino group, a dibenzylamino group, an N-ethyl-N-methylamino group, an N-methyl-N-propylamino group, an N-isopropyl-N-methylamino group, an N-butyl-N-methylamino group, an N-sec-butyl-N-methylamino group, an N-tert-butyl-N-methylamino group, an N-benzyl-N-methylamino group, an N-ethyl-N-propylamino group, an N-ethyl-N-isopropylamino group, an N-butyl-N-ethylamino group, an N-sec-butyl-N-ethylamino group, an N-tert-butyl-N-ethylamino group, an N-benzyl-N-ethylamino group, an N-isopropyl-N-propylamino group, an N-butyl-N-propylamino group, an N-sec-butyl-N-propylamino group, an N-tert-butyl-N-propylamino group, an N-benzyl-N-propylamino group, an N-butyl-N-isopropylamino group, an N-sec-butyl-N-isopropylamino group, an N-tert-butyl-N-isopropylamino group, an N-benzyl-N-isopropylamino group, an N-butyl-N-sec-butylamino group, an N-butyl-N-tert-butylamino group, an N-butyl-N-benzylamino group, an N-benzyl-N-sec-butylamio group, an N-benzyl-N-tert-butylamio group, an N-sec-butyl-N-tert-butylamino group, etc. are exemplified.

A 6 to 10-membered monocyclic or fused polycyclic aryl group of the present invention indicates an aromatic cyclic group composed of 6 to 10 carbons only, which indicates a phenyl group, naphthyl group, etc.

A 5 to 10-membered monocyclic or fused polycyclic hetero aryl group of the present invention indicates an aromatic hetero ring composed of 5 to 10 atoms having one or more hetero atoms which are selected from the group consisting of an oxygen atom, a nitrogen atom, and a sulfur atom, etc., and are, for example,
a thiazolyl group, a thienyl group, a furyl group, an isothiazolyl group, an oxazolyl group, an isoxazolyl group, a pyrrolyl group, an imidazolyl group, a furazanyl group, a pyrazolyl group, a pyridyl group, a pyrazinyl group, a pyrimidinyl group, a pyridazinyl group, a triazinyl group, a quinolyl group, an isoquinolyl group, an indolyl group, an isoindolyl group, an indolizinyl group, an indazolyl group, a phthalazinyl group, a naphthyridinyl group, a quinoxalinyl group, a quinazolinyl group, a purinyl group, a pteridinyl group, a cinnolinyl group, a chromenyl group, a benzofuranyl group, a benzoxazolyl group, a benzisoxazolyl group, a benzothiazolyl group, a benzisothiazolyl group, a benzimidazolyl group, etc. are exemplified.

A 5 to 6-membered monocyclic heteroaryl group of the present invention indicates a monocyclic aromatic hetero ring group composed of 5 to 6 atoms having one or more hetero atoms which are selected from the group consisting of an oxygen atom, a nitrogen atom, and a sulfur atom, etc., and are, for example,
a thiazolyl group, a thienyl group, a furyl group, an isothiazolyl group, an oxazolyl group, an isoxazolyl group, a pyrrolyl group, an imidazolyl group, a furazanyl group, a pyrazolyl group, a pyridyl group, a pyrazinyl group, a pyrimidinyl group, a pyridazinyl group, a triazinyl group, etc. are exemplified.

A 3 to 10-membered monocyclic or fused polycyclic nonaromatic hetero ring with one or more hetero atoms of the present invention indicates a monocyclic or fused polycyclic nonaromatic cyclic group composed of 3 to 10 atoms having one or more hetero atoms which are selected from the group consisting of an oxygen atom, a nitrogen atom, and a sulfur atom, etc., and are, for example,
an oxiranyl group, an aziridinyl group, an oxetanyl group, a thietanyl group, a thiolanyl group, a thiomorpholinyl group, a tetrahydrofuryl group, a pyrrolidinyl group, a pyrrolinyl group, an imidazolidinyl group, an imidazolinyl group, a pyrazolidinyl group, a pyrazolinyl group, a pyranyl group, a tetrahydropyranyl group, a piperazinyl group, a morpholinyl group, a piperidinyl group, an indolinyl group, an isoindolinyl group, a dihydrobenzofuranyl group, a tetrahydroquinolyl group, a tetrahydroisoquinolyl group, etc. are exemplified.

A 3 to 7-membered monocyclic nonaromatic hetero ring with one or more hetero atoms of the present invention indicates a monocyclic or fused polycyclic nonaromatic cyclic group composed of 3 to 7 atoms having one or more hetero atoms which are selected from the group consisting of an oxygen atom, a nitrogen atom, and a sulfur atom, etc., and are, for example,
an oxiranyl group, an aziridinyl group, an oxetanyl group, a thietanyl group, a thiolanyl group, a thiomorpholinyl group, a tetrahydrofuryl group, a pyrrolidinyl group, a pyrrolinyl group, an imidazolidinyl group, an imidazolinyl group, a pyrazolidinyl group, a pyrazolinyl group, a pyranyl group, a tetrahydropyranyl group, a piperazinyl group, a morpholinyl group, a piperidinyl group, etc. are exemplified.

A monocyclic or fused polycyclic hetero ring of the present invention indicates a monocyclic or fused polycyclic heteroaryl group having one or more hetero atoms and a monocyclic or fused polycyclic nonaromatic hetero ring having one or more hetero atoms, and, for example,
an oxiranyl group, an aziridinyl group, an oxetanyl group, a thietanyl group, a thiolanyl group, a thiomorpholinyl group, a tetrahydrofuryl group, a pyrrolidinyl group, a pyrrolinyl group, an imidazolidinyl group, an imidazolinyl group, a pyrazolidinyl group, a pyrazolinyl group, a pyranyl group, a tetrahydropyranyl group, a piperazinyl group, a morpholinyl group, a piperidinyl group, an indolinyl group, an isoindolinyl group, a dihydrobenzofuranyl group, a thiazolyl group, a thienyl group, a furyl group, an isothiazolyl group, an oxazolyl group, an isoxazolyl group, a pyrrolyl group, an imidazolyl group, a furazanyl group, a pyrazolyl group, a pyridyl group, a pyrazinyl group, a pyrimidinyl group, a pyridazinyl group, a triazinyl group, a quinolyl group, an isoquinolyl group, an indolyl group, an isoindolyl group, an indolizinyl group, an indazolyl group, a phthalazinyl group, a naphthyridinyl group, a quinoxalinyl group, a quinazolinyl group, a purinyl group, a pteridinyl group, a cinnolinyl group, a chromenyl group, a benzofuranyl group, a benzoxazolyl group, a benzisoxazolyl group, a benzothiazolyl group, a benzisothiazolyl group, a benzimidazolyl group, a tetrahydroquinolyl group, a tetrahydroisoquinolyl group, etc. are exemplified.

A compound of formula (I) (also referred to as a compound (I)) can exist in the form of tautomers represented by the following formula (I₁) and (I₂):

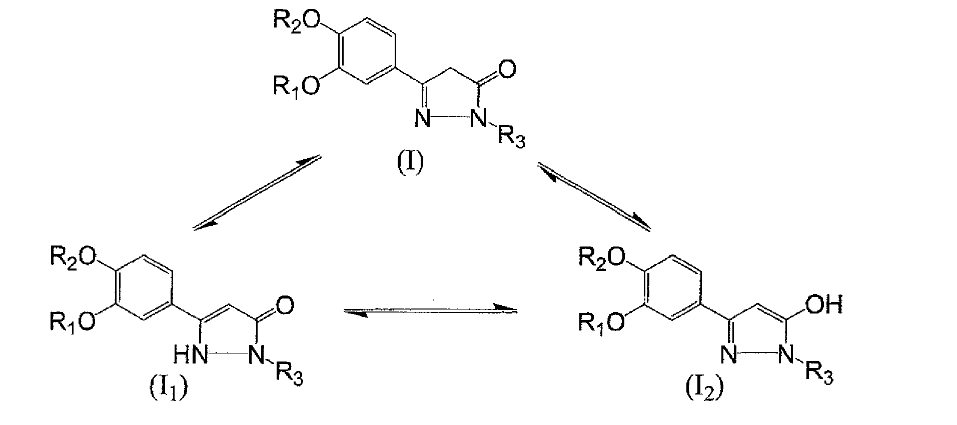

Hereinafter, a compound of the present invention can be represented or referred to by either tautomeric form (I). And it should be discerned that each tautomeric and any forms of formula (I), specifically formula (I₁) and (I₂), are comprised in the present invention.

Some compounds of the above formula (I) have an asymmetrical carbon atom, and thereby optical isomers exist. These optical isomers are also comprised in the present invention. In addition, a salt of the compounds of the above formula (I) and optical isomers thereof is also comprised in the present invention, and the salt is, preferably, a pharmacologically acceptable salt; for example, an inorganic salt such as hydrochloride, hydrobromate, hydroiodide, and phosphate; and an organic salt such as oxalate, maleate, fumarate, lactate, malate, citrate, tartrate, benzoate, methanesulfonate, and p-toluenesulfonate are exemplified.

Furthermore, a hydrate or a solvate of the compounds of the above formula (I), tautomers thereof, and the salts thereof are also comprised in the present invention, and regarding the solvent for the solvate, methanol, ethanol, isopropanol, butanol, acetone, ethyl acetate, chloroform, etc. are exemplified.

Although a compound of the above formula (I) can be manufactured by a publicly known method (Journal of Medicinal Chemistry, 19 (2), 239-43 (1976), Journal of Medicinal Chemistry, 30 (10), 1724-8 (1987), etc.), an example of the manufacturing method is explained by the following reaction schemes.

### Preparation Method 1

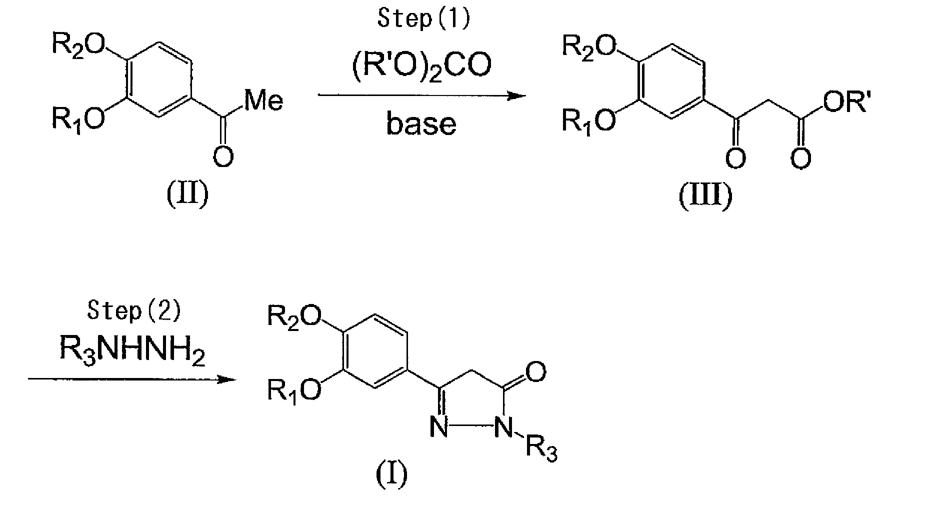

### Step (I):

Dialkyl carbonate is subjected to the reaction with acetophenone (II) in the presence of a base, leading to a β-ketoester form (III). This reaction is carried out either in the absence or presence of a solvent. Any solvent is available as far as it does not inhibit the reaction; for example, benzene, toluene, xylene, tetrahydrofuran, dimethylformamide, etc. are exemplified. The reaction temperature is generally between 0 to 150°C. The compound obtained by this reaction is purified by a publicly known method such as crystallization, recrystallization, chromatography, etc.

### Step (2):

A hydrazine derivative is subjected to the reaction with the β-ketoester form (III), leading to a 5-phenylpyrazolone form (I). This reaction is carried out either in the absence or presence of a solvent. Any solvent is available as far as it does not inhibit the reaction; for example, benzene, toluene, xylene, tetrahydrofuran, dimethylformamide, ethanol, acetate, water, etc. are exemplified. The reaction temperature is generally between 0 to 120°C. The compound obtained by this reaction is purified by a publicly known method such as crystallization, recrystallization, chromatography, etc.

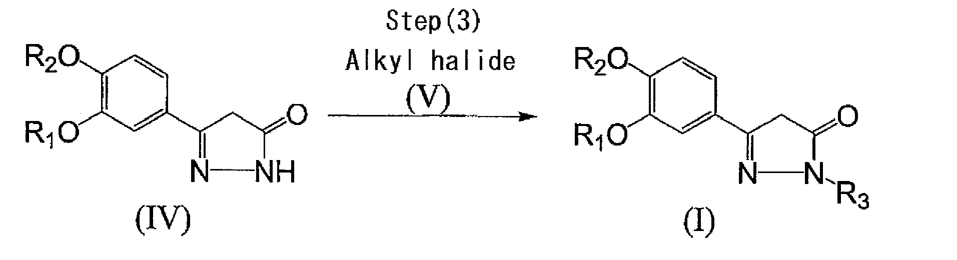

### Step (3):

In the case R₃ is hydrogen, alkyl halides (V) are subjected to the reaction in the presence of a base, synthesizing the compound (I). Regarding the base used for this reaction, for example, sodium hydroxide, potassium hydroxide, sodium hydride, potassium carbonate, etc. are exemplified. This reaction is carried out either in the absence or presence of a solvent. Any solvent is available as far as it does not inhibit the reaction; for example, benzene, toluene, xylene, tetrahydrofuran, dimethylformamide, etc. are exemplified. The compound obtained by this reaction is purified by a publicly known method.

The starting material used in the above reaction step is synthesized from a compound which is commercially available or well-known based on a publicly known method. The starting material, the ketone derivative (II), can be prepared by the method described in WO94/10118.

In the case the compound of the present invention is used as a therapeutic agent, it is administered by itself or as a composite with a pharmaceutically available carrier. The composition is determined based on the solubility of the compound, the chemical nature, the administering route, the plan for administration, etc.

For example, oral administration may be carried out in a dosage form such as granules, powder, tablets, pellets, hard capsules, soft capsules, syrup, emulsion, suspension, liquid, etc., or parenteral administration in a dosage form such as injectable solutions (intravenous, intramuscular, and subcutaneous), ointment, suppositories, aerosol, etc. Furthermore, an injectable solution may be prepared from powders when used. Together with the compound of the present invention, a carrier or a diluent of an organic or inorganic solid or liquid for medicinal use suitable for an oral, enteral, parenteral, or topical administration can be employed. For example, in the case of oral administration, a desirable administration dosage form can be prepared using a vehicle such as lactose, glucose, cornstarch, and sucrose; a disintegrant such as calcium carboxymethyl cellulose and hydroxypropyl cellulose; a lubricant such as calcium stearate, magnesium stearate, talc, polyethylene glycol, and hardened oil; a wetting agent such as hydroxypropyl cellulose, hydroxypropyl methyl cellulose, carboxymethyl cellulose, polyvinyl alcohol, gelatin, and gum arabic; and in addition, a detergent and a flavoring substance according to need.

Moreover, in the case of parental administration, a diluent such as water, ethanol, glycerin, propylene glycol, polyethylene glycol, agar, and tragacanth gum can be used, and, according to need, a solubilizing agent, a buffering agent, a preserving agent, fragrance, and a coloring agent can be also used. The preparation method of the drug formulation can depend on a conventional method.

In the case of oral administration as a compound of the present invention, the clinically applied dose for an adult is generally 0.01 to 1,000 mg a day, and preferably, 0.01 to 100 mg, but it is further preferable to appropriately adjust the dose based on the age, the disease condition, the symptoms, the presence or absence of coadministration, etc. Said daily dosage of the pharmaceutical agent (a compound of the present invention) can be administered once a day, or two or three times a day at adequate intervals, or can be administered intermittently. In addition, in the case of injection as a compound of the present invention for an adult, it is preferable to administer a dose of 0.001 to 100 mg continuously or intermittently.

### Examples

The present invention is specifically explained below based on examples and experimental examples, but the present invention is not limited to the following examples or experimental examples as far as it does not extend beyond the scope of the present invention.

Elution of the column chromatography of the examples was carried out under observation by TLC (Thin Layer Chromatography). The TLC observation utilized the 60F254 prepared by Merck & Co., Inc. for TLC plates, the solvent used as the elution solvent in the column chromatography for a developing solvent, and a UV detector was used in the detection method. The silica gel PSQ60B or PSQ100B prepared by Fuji Silysia Chemical Ltd. was used as the silica gel (SiO₂) for a column. NMR spectra were recorded on a JNM-AL400 prepared by JEOL Datum Ltd. using tetramethylsilane as the internal or external standard. A chemical shift is represented by a δ-value, and a coupling constant is represented by Hz. The numerical value shown in a parenthsis for a mixed solvent represents a mix ratio of each solvent by volume. In addition, the percentage in a solution represents the number of grams in a 100 ml solution. Furthermore, the symbols in the examples mean the following.
s : singlet
d : doublet
t : triplet
dd : double doublet
m : multiplet
br s : broad singlet
J : coupling constant

### Example 1: Synthesis of 3-(3-(2-indanyloxy)-4-methoxyphenyl)-5-pyrazolone (Compound No.1 of Table 1)

### (1) Synthesis of ethyl 3-(3-(2-indanyloxy)-4-methoxyphenyl)-3-oxopropanoate

3-(2-indanyloxy)-4-methoxyacetophenone was suspended in diethyl carbonate, and sodium hydride was added thereto. The reaction solution was stirred at 120°C for an hour, and then poured into ice water. The solution was extracted with ethyl acetate and was then washed with brine, and dried over anhydrous magnesium sulfate. After filtration and condensation, the residue was purified by flash chromatography (SiO₂: eluted with 30% ethyl acetate-hexane), and the title compound was obtained.

### (2) Synthesis of 3-(3-(2-indanyloxy)-4-methoxyphenyl)-1-methyl-5-pyrazolone

Ethyl 3-(3-(2-indanyloxy)-4-methoxyphenyl)-3-oxopropanoate and hydrazine monohydrate were dissolved in ethanol and was heated to reflux for 3 hrs. The precipitate was subjected to suction filtration and dried, and thereby the title compound which is a colorless crystal was obtained.
¹H-NMR (DMSO-D₆) δ: 7.31 (1H, d, J = 1.8 Hz), 7.28-7.24 (2H, m), 7.20 (1H, dd, J = 8.4, 1.9 Hz), 7.18-7.15 (2H, m), 6.96 (1H, d, J = 8.4 Hz), 5.83 (1H, s), 5.27-5.23 (1H, m), 3.70 (3H, s), 3.38 (2H, dd, J = 17.5, 6.3 Hz), 3.35 (2H, s), 3.03 (2H, dd, J = 16.8, 2.4 Hz).

### Example 2: Synthesis of 3-(3-(2-indanyloxy)-4-methoxyphenyl.)-l-methyl-5-pyrazolone (Compound No.2 of Table 1)

By using a similar method to Example 1 (2), with the exception that methylhydrazine was used in place of hydrazine monohydrate, the title colorless amorphous compound was obtained.
¹H-NMR (CDCl₃) δ: 7.41 (1H, d, J = 2.0 Hz), 7.26-7.18 (4H, m), 7.08 (1H, dd, J = 8.4, 2.0 Hz), 6.87 (1H, d, J = 8.4 Hz), 5.30-5.25 (1H, m), 3.85 (3H, s), 3.58 (2H, s), 3.44 (2H, dd, J = 16.7, 6.7 Hz), 3.40 (3H, s), 3.26 (2H, dd, J = 16.6, 3.8 Hz),

### Example 3: Synthesis of 3-(3-(cyclopentyloxy)-4-methoxyphenyl)-5-pyrazolone (Compound No.3 of Table 1)

By using a similar method to Example 1 (1) to (2), with the exception that 3-(cyclopentyloxy)-4-methoxyacetophenone was used in place of 3-(2-indanyloxy)-4-methoxyacetophenone, the title colorless solid compound was obtained.
¹H-NMR (DMSO-D₆) δ: 7.20 (1H, s), 7.16 (1H, d, J = 8.3 Hz), 6.95 (1H, d, J = 8.3 Hz), 5.79 (1H, s), 4.86-4.81 (1H, m), 3.74 (3H, s), 3.34 (2H, s), 1.91-1.57 (8H, m).

### Example 4: Synthesis of 3-(3-(cyclopentyloxy)-4-methoxyphenyl)-l-methyl-5-pyrazolone (Compound No.4 of Table 1)

By using a similar method to Example 1 (1) to (2), with the exception that 3-(cyclopentyloxy)-4-methoxyacetophenone and methylhydrazine were used in place of 3-(2-indanyloxy)-4-methoxyacetophenone and hydrazine monohydrate, respectively, the title light yellow solid compound was obtained.
¹H-NMR (CDCl₃) δ: 7.33 (1H, d, J = 2.0 Hz), 7.05 (1H, dd, J = 8.4, 2.0 Hz), 6.85 (1H, dd, J = 8.4 Hz), 4.88-4.84 (1H, m), 3.88 (3H, s), 3.57 (2H, s), 3.39 (3H, s), 2.02-1.83 (6H, m), 1.66-1.59 (2H, m).

### Example 5: Synthesis of 3-(3-(2-indanyloxy)-4-methoxyphenyl)-l-phenyl-5-pyrazolone (Compound No.5 of Table 1)

By using a similar method to Example 1 (1) to (2), with the exception that phenylhydrazine was used in place of hydrazine monohydrate, the title colorless solid compound was obtained.
¹H-NMR (DMSO-D₆) δ: 11.74 (1H, br, s), 7.80 (2H, d, J = 7.8 Hz), 7.49-7.41 (3H, m), 7.34 (1H, dd, J = 8.3, 1.8 Hz), 7.28-7.15 (5H, m), 6.98 (1H, d, J = 8.3 Hz), 5.96 (1H, s), 5.31-5.26 (1H, m), 3.71 (3H, s), 3.40-3.32 (2H, m), 3.06 (2H, dd, J = 16.9, 2.1 Hz).

### Example 6: Synthesis of 3-(3-(cyclopentyloxy)-4-methoxyphenyl)-l-phenyl-5-pyrazolone (Compound No.6 of Table 1)

By using a similar method to Example 1 (1) to (2), with the exception that 3-(cyclopentyloxy)-4-methoxyacetophenone and phenylhydrazine were used in place of 3-(2-indanyloxy)-4-methoxyacetophenone and hydrazine monohydrate, respectively, the title colorless solid compound was obtained.
¹H-NMR (CDCl₃) δ: 8.00-7.95 (2H, m), 7.46-7.41 (3H, m), 7.24-7.19 (1H, m), 7.15 (1H, dd, J = 8.3, 2.0 Hz), 6.89 (1H, d, J = 8.3 Hz), 4.91-4.87 (1H, m), 3.90 (3H, s), 3.83 (2H, s), 2.05-1.82 (6H, m), 1.69-1.62 (2H, m).

### Example 7: Synthesis of 3-(3-(cyclopentyloxy)-4-methoxyphenyl)-1-(4-hydroxycarbonylphenyl)-5-pyrazolone (Compound No.7 of Table 1)

By using a similar method to Example 1 (1) to (2), with the exception that 3-(cyclopentyloxy)-4-methoxyacetophenone and 4-hydrazinobenzoic acid were used in place of 3-(2-indanyloxy)-4-methoxyacetophenone and hydrazine monohydrate, respectively, the title yellow solid compound was obtained.
¹H-NMR (CDCl₃) δ: 8.20-8. 14 (4H, m), 7.46 (1H, d, J = 2.0 Hz), 7.18 (1H, dd, J = 8.4, 2.0 Hz), 6.90 (1H, d, J = 8.4 Hz), 4.93-4.88 (1H, m), 3.91 (3H, s), 3.88 (2H, s), 2.08-1.83 (6H, m), 1.70-1.64 (2H, m).

### Example 8: Synthesis of 3-(3-(cyclopentyloxy)-4-(difluoromethoxy)phenyl) -5-pyrazolone (Compound No.8 of Table 1)

By using a similar method to Example 1 (1) to (2), with the exception that 3-(cyclopentyloxy)-4-(difluoromethoxy)acetophenone was used in place of 3-(2-indanyloxy)-4-methoxyacetophenone, the title compound was obtained.
¹H-NMR (DMSO-D₆) δ: 12.06 (1H, s), 7.39 (1H, d, J = 1.8 Hz), 7.22-7.14 (2H, m), 6.98 (1H, t, J = 74.8 Hz), 5.90 (1H, s), 4.96-4.92 (1H, m), 1.97-1.56 (8H, m).

### Example 9: Synthesis of 3-(3-(cyclopropylmethoxy)-4-methoxyphenyl)-5- pyrazolone (Compound No.9 of Table 1)

By using a similar method to Example 1 (1) to (2), with the exception that 3-(cyclopropylmethoxy)-4-methoxyacetophenone was used in place of 3-(2-indanyloxy)-4-methoxyacetophenone, the title compound was obtained.
¹H-NMR (ACETONE-D₆) δ: 7.28 (1H, d, J = 2.1 Hz), 7.23 (1H, dd, J = 8.4, 2.1 Hz), 6.98 (1H, d, J = 8.4 Hz), 5.86 (1H, s), 3.89 (2H, d, J = 6.8 Hz), 3.83 (3H, s), 2.84-2.79 (2H, m), 1.31-1.24 (1H, m), 0.62-0.56 (2H, m), 0.37-0.32 (2H, m).

### Example 10: Synthesis of 3-(3-(3-tetrahydrofuranyloxy)-4-methoxyphenyl)-5- pyrazolone (Compound No.10 of Table 1)

By using a similar method to Example 1 (1) to (2), with the exception that 3-(3-tetrahydrofuranyloxy)-4-methoxyacetophenone was used in place of 3-(2-indanyloxy)-4-methoxyacetophenone, the title compound was obtained.
¹H-NMR (ACETONE-D₆) δ: 7.29-7.26 (2H, m), 7.02 (1H, d, J = 8.1 Hz), 5.87 (1H, s), 5.11-5.07 (1H, m), 3.96-3.77 (4H, m), 3.83 (3H, s), 2.83-2.79 (2H, m), 2.26-2.05 (2H, m).

### Example 11: Synthesis of 1-cyclopentyl-3-(3-(cyclopentyloxy)-4-methoxyphenyl)-5-pyrazolone (Compound No.11 of Table 1)

3-(3-(cyclopentyloxy)-4-methoxyphenyl)-5-pyrazolone produced in Example 3 was dissolved in dimethyl formamide, and sodium hydride was added thereto, and then the solution was stirred for an hour. Subsequently, bromocyclopentane was added to the solution and further stirred for 18 hrs, and then the reaction solution was poured into ice water. The solution was extracted with ethyl acetate, which was then washed with brine, and dried over anhydrous magnesium sulfate. After filtration and condensation, the residue was purified by the flash chromatography (SiO₂: eluted with 20% ethyl acetate-hexane), and the title light brown amorphous compound was obtained.
¹H-NMR (CDCl₃) δ: 7.08-7.03 (2H, m), 6.89 (1H, d, J = 8.2 Hz), 5.84 (1H, s), 4.99-4.95 (1H, m), 4.84-4.79 (1H, m), 3.87 (3H, s), 1.98-1.57 (16H, m).

### Example 12: Synthesis of 3-(4-methoxy-3-(3-pentyloxy)phenyl)-5-pyrazolone (Compound No.12 of Table 1)

By using a similar method to Example 1 (1) to (2), with the exception that 4-methoxy-3-(3-pentyloxy)acetophenone was used in place of 3-(2-indanyloxy)-4-methoxyacetophenone, the title compound was obtained.
¹H-NMR (CDCl₃) δ: 8.55 (1H, s), 7.33 (1H, s), 7.08-7.02 (1H, m), 6.87 (1H, d, J = 8.3 Hz), 4.21-4.13 (1H, m), 3.89 (3H, s), 3.55 (2H, s), 1.79-1.66 (4H, m), 0.98 (6H, t, J = 7.2 Hz).

### Example 13: Synthesis of 3-(3-(cyclopentyloxy)-4-methoxyphenyl)-1-(2-(dibenzylamino)ethyl)-5-pyrazolone (Compound No.13 of Table 1)

By using the method of Example 11, with the exception that N,N-dibenzyl-2-chloroethanolamine hydrochloride was used in place of bromocyclopentane, the title light brown amorphous compound was obtained.
¹H-NMR (CDCl₃) δ: 7.39 (4H, d, J = 7.1 Hz), 7.32-7.28 (4H, m), 7.25-7.20 (2H, m), 7.04 (1H, dd, J = 8.3, 1.8 Hz), 7.00 (1H, d, J = 1.8 Hz), 6.89 (1H, d, J = 8.3 Hz), 5.81 (1H, s), 4.84-4.79 (1H, m), 4.29 (2H, t, J = 6.0 Hz), 3.87 (3H, s), 3.72 (4H, s), 2.90 (2H, t, J = 6.0 Hz), 1.96-1.82 (6H, m), 1.65-1.58 (2H, m).

### Example 14: Synthesis of 3-(3-(cyclopentyloxy)-4-methoxyphenyl)-1-((1-methylpiperidin-3-yl)methyl)-5-pyrazolone (Compound No.14 of Table 1)

By using the method of Example 11, with the exception that 3-(chloromethyl)-1-methylpiperidine hydrochloride was used in place of bromocyclopentane, the title light brown amorphous compound was obtained.
¹H-NMR (CDCl₃) δ: 7.07-7.01 (2H, m), 6.89 (1H, d, J = 8.2 Hz), 5.86 (1H, s), 4.84-4.79 (1H, m), 4.07 (1H, dd, J = 9.8, 5.7 Hz), 4.00 (1H, dd, J = 9.8, 7.4 Hz), 3.87 (3H, s), 2.97 (1H, d, J = 9.8 Hz), 2.78 (1H, d, J = 11.4 Hz), 2.28 (3H, s), 2.19-2.12 (1H, m), 1.96-1.59 (14H, m).

### Example 15: Synthesis of 3-(3-(cyclopentyloxy)-4-methoxyphenyl)-1-(hydroxycarbonylmethyl)-5-pyrazolone (Compound No.15 of Table 1)

By using the method of Example 11, with the exception that bromoacetic acid was used in place of bromocyclopentane, the title compound was obtained.
¹H-NMR (DMSO-D₆) δ: 12.26 (1H, s), 8.30 (1H, s), 7.23-7.18 (2H, m), 6.98 (1H, d, J = 7.8 Hz), 6.07 (1H, s), 4.87-4.83 (1H, m), 4.65 (2H, s), 3.76 (3H, s), 1.95-1.87 (2H, m), 1.75-1.54 (6H, m).

### Example 16: Synthesis of 3-(4-(cyclopentyloxy)-3-methoxyphenyl)-5-pyrazolone (Compound No.16 of Table 1)

By using a similar method to Example 1 (1) to (2), with the exception that 3-(4-cyclopentyloxy)-3-methoxyacetophenone was used in place of 3-(2-indanyloxy)-4-methoxyacetophenone, the title compound was obtained.
¹H-NMR (DMSO-D₆) δ: 11.88 (1H, br, s), 9.59 (1H, br, s), 7.23 (1H, d, J = 2.0 Hz), 7.14 (1H, dd, J = 8.3, 2.0 Hz), 6.92 (1H, d, J = 8.3 Hz), 5.79 (1H, s), 4.81-4.76 (1H, m), 3.78 (3H, s), 1.92-1.53 (8H, m).

**Table 1**

| | | | |
|---|---|---|---|
| 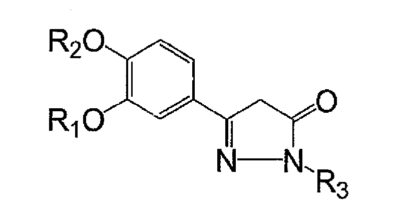 | | | |

| Compound No. | R₁ | R₂ | R₃ |
|---|---|---|---|
| 1 | 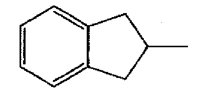 | Me | H |
| 2 | 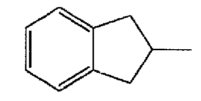 | Me | Me |
| 3 | 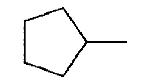 | Me | H |
| 4 | 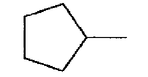 | Me | Me |
| 5 | 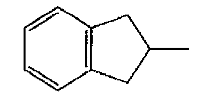 | Me | Ph |
| 6 | 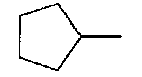 | Me | Ph |
| 7 | 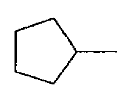 | Me | 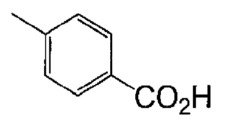 |
| 8 | 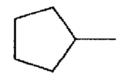 | CHF₂ | H |
| 9 | 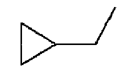 | Me | H |
| 10 | 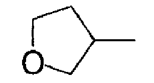 | Me | H |
| 11 | 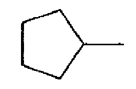 | Me | 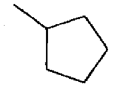 |
| 12 | 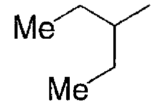 | Me | H |
| 13 | 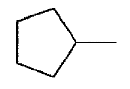 | Me | 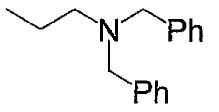 |
| 14 | 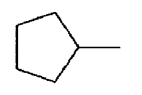 | Me | 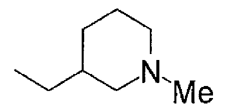 |
| 15 | 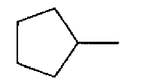 | Me | 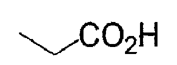 |
| 16 | Me | 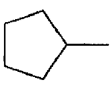 | H |

### Example 17: Preparing of Tablets

30 g of 3-(3-(cyclopentyloxy)-4-methoxyphenyl)-5-pyrazolone (Compound No. 3 of Table 1), 253 g of lactose, 63 g of cornstarch, 40 g of low substituted hydroxypropyl cellulose, and 4 g of calcium stearate were mixed, which was then compressed by an ordinary method so that each tablet contains 10 mg of said compound.

### Example 18: Preparing of Capsules

30 g of 3-(3-(cyclopentyloxy)-4-methoxyphenyl)-5-pyrazolone (Compound No. 3 of Table 1), 260 g of lactose, 66 g of cornstarch, and 4 g of calcium stearate were mixed, which was then filled in gelatin capsules by a usual method so that each capsule contains 10 mg of said compound.

### Example of Biological Experiment 1:

### PAI-1 production inhibitory activity using bronchial epithelial cells

The effect of a compound against PAI-1 production activity was investigated using bronchial epithelial cells which reflect the PAI-1 concentration in the lung tissue involved in fibrosis (lung).

The effect against the amount of PAI-1 production was investigated using human bronchial epithelial cells (BEAS-2B). 0.1 ml of the BEAS-2B cells prepared to 2 x 10⁵ cells/ml were added to each of the 96 wells of a collagen I coated plate, and precultured for 24 hrs. Then 0.1 ml of a medium containing 10 µM of TGF-β was added, and after 24 hrs of incubation, the PAI-1 amount in the supernatant of the medium was measured by an ELISA kit. The materials to be tested were, after dissolved in dimethyl sulfoxide (the final concentration: 0.1% or lower), prepared to 0.2, 1, 5, and 25 µM with a phosphate buffer solution, and added to the preculture. The PAI-1 production inhibitory activity of a compound of the present invention is represented by an IC₅₀ value, and shown in Table 2 below.

**Table 2**

| Compound No. | The PAI-1 production inhibitory activity IC50 (µM) |
|---|---|
| 2 | 1.42 |
| 3 | 7.59 |
| 6 | 2.56 |

### Example of Biological Experiment 2:

### Activity to the bleomycin-induced lung fibrosis model

It is reported that administering bleomycin into the trachea induces fibrosis of the lung. According to the pathological observation, it is similar to clinical idiopathic pulmonary fibrosis in terms of the thickened fibrosis of the alveolar wall, an increase in inflammatory cells, an increment in the amount of hydroxyproline, etc., and is often used as a lung fibrosis experimental model (Biochem. Biophys. Res. Comm., 288, 747 (2001)). Bleomycin hydrochloride (Bleo: Nippon Kayaku Co., Ltd.) dissolved in physiological saline was injected with 500 µl of compressed air into the bronchi of C57BL/6 mice (6 week old, female) at 6 mg/kg and 25 µl/body. Three weeks after the intratracheal administration, the amount of hydroxyproline in the lung was measured. The materials to be tested, at 0.3 mg/kg per dose, were suspended in 0.5% sodium carboxymethyl cellulose, and administered orally once a day. The effect of the present invention is represented by a inhibitory ratio against the increment in the amount of lung hydroxyproline of the solvent-administered group, and is shown in Table 3 below.

**Table 3**

| Compound No. | Inhibitory ratio against the increment in the lung hydroxyproline amount (%) |
|---|---|
| 1 | 77 |
| 2 | 75 |
| 3 | 73 |
| 4 | 73 |
| 5 | 75 |
| 6 | 52 |
| 7 | 57 |
| 8 | 61 |
| 9 | 60 |

### Example of Biological Experiment 3: Acute toxicity

Compounds No.1 to No. 16 of the present invention were suspended in physiological saline containing 0.5% sodium carboxymethyl cellulose, and administered intraperitoneally to ddY male mice. The viability was observed on the following day, and no compound showed a fatal case at the administered amount of 30 mg/kg.

### Industrial applicability

The compound of the present invention has an excellent PAI-1 production inhibitory activity, and is effective by the fibrinolytic activity for preventing and/or treating tissue fibrotic diseases such as lung fibrosis and kidney fibrosis, and diseases of which a pathological thrombus becomes the cause, such as ischemic cardiac diseases (cardiac infarction and angina pectoris), atrial thrombus, lung embolism, venous thromboembolism, etc.

## Claims

1. A 5-phenyl-3-pyrazolone derivative represented by the following formula (I): 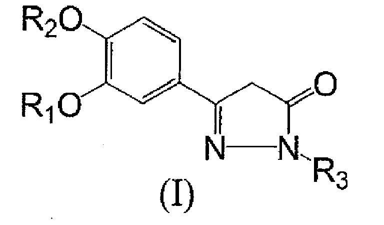 wherein R₁ represents a C₁ to C₈ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or fused polycyclic aryl group; or a monocyclic or fused polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more of said substituents, or an indanyl group; R₂ represents a C₁ to C₄ alkyl group, a C₁ to C₄ haloalkyl group, or a C₃ to C₇ cycloalkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more of an alkyl group on the nitrogen atom; an alkoxy group; an alkoxycarbonyl group; an acyl group; a monocyclic or fused polycyclic aryl group; or a monocyclic or fused polycyclic hetero ring having one or more hetero atoms); and R₃ represents a hydrogen atom or a C₁ to C₅ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or fused polycyclic aryl group; or a monocyclic or fused polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more substituents (a methyl group; a hydroxyl group; or a carboxyl group), a monocyclic or fused polycyclic aryl group which may have one or more substituents (a methyl group; a hydroxyl group; or a carboxyl group), or a monocyclic or fused polycyclic hetero aryl group having one or more hetero atoms; or an optical isomer, a pharmaceutically acceptable salt thereof, or a hydrate or solvate thereof.

2. A compound described in claim 1, wherein R₁ represents a C₁ to C₈ alkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to C₆ acyl group; a 6 to 10-membered monocyclic or fused polycyclic aryl group; a 3 to 10-membered monocyclic or fused polycyclic nonaromatic hetero ring with one or more hetero atoms; or a 5 to 10-membered monocyclic or fused polycyclic hetero aryl group with one or more hetero atoms), a C₃ to C₇ cycloalkyl group which may have one or more of said substituents, a 3 to 7-membered monocyclic nonaromatic hetero ring with one or more hetero atoms which may have one or more of said substituents, or an indanyl group; R₂ represents a C₁ to C₄ alkyl group, a C₁ to C₄ haloalkyl group, or a C₃ to C₇ cycloalkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to C₆ acyl group; a 6 to 10-membered monocyclic or fused polycyclic aryl group; a 3 to 10-membered monocyclic or fused polycyclic nonaromatic hetero ring with one or more hetero atoms; or a 5 to 10-membered monocyclic or fused polycyclic hetero aryl group with one or more hetero atoms); and R₃ represents a hydrogen atom, a C₁ to C₅ alkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to C₆ acyl group; a 6 to 10-membered monocyclic or fused polycyclic aryl group; a 3 to 10-membered monocyclic or fused polycyclic nonaromatic hetero ring with one or more hetero atoms; or a 5 to 10-membered monocyclic or fused polycyclic hetero aryl group with one or more hetero atoms), a C₃ to C₇ cycloalkyl group which may have one or more of said substituents, a 3 to 7-membered monocyclic nonaromatic hetero ring with one or more hetero atoms which may have one or more of said substituents, a 6 to 10-membered monocyclic or fused polycyclic aryl group which may have one or more substituents (a methyl group; a hydroxyl group; or a carboxyl group), or a 5 to 10-membered monocyclic or fused polycyclic heteroaryl group having one or more hetero atoms.

3. A compound described in claim 1 or 2, wherein R₁ is a C₁ to C₈ alkyl group, a C₁ to C₅ alkyl group having one or more phenyl or furyl groups as substituents, a C₁ to C₅ alkyl group with one or more C₃ to C₇ cycloalkyl groups, as substituents, which may have one or more phenyl or C₁ to C₃ alkyl groups as substituents, a C₄ to C₆ cycloalkyl group which may have one or more substituents (a methyl group; hydroxyl group; a carboxyl group), a 5 to 6-membered monocyclic nonaromatic hetero ring with one or more hetero atoms which may have one or more substituents (a methyl group; hydroxyl group; a carboxyl group), or an indanyl group; R₂ is a methyl group, an ethyl group, a trifluoromethyl group, a difluoromethyl group, a fluoromethyl group, or a C₄ to C₆ cycloalkyl group having one or more substituents (a methyl group; a hydroxyl group; a carboxyl group); and R₃ is a hydrogen atom, a C₁ to C₃ alkyl group which may have a substituent (a C₁ to C₃ alkyl group; a carboxyl group; an amino group which may have one or more C₁ to C₄ alkyl or benzyl groups on the nitrogen atom; a piperidine group which may have one or more C₁ to C₄ alkyl or benzyl groups on the nitrogen atom; a phenyl group), a phenyl group which may have one or more substituents (a methyl group; a hydroxyl group; a carboxyl group), a C₄ to C₆ cycloalkyl group which may have one or more substituents (a methyl group; a hydroxyl group; a carboxyl group).

4. A compound described in any one of claims 1 to 3, wherein R₁ is a methyl group, an ethyl group, a 3-pentyl group, a C₁ to C₅ alkyl group having a phenyl group as a substituent, a cyclopentyl group, a cyclopropylmethyl group, a (1-methylcyclopropyl) methyl group, a (1-phenylcyclopropyl) methyl group, a 3-tetrahydrofuryl group, a 2-ethylbutyl group, or an 2-indanyl group; R₂ is a methyl group, an ethyl group, a difluoromethyl group, or a cyclopentyl group; and R₃ is a hydrogen atom, a methyl group, an ethyl group, a cyclopentyl group, a phenyl group, a 4-hydroxycarbonylphenyl group, a benzyl group, a phenethyl group, a 2-(dibenzylamino)ethyl group, a hydroxycarbonylmethyl group, or a (1-methylpiperidine-3-yl)methyl group.

5. A medical drug for preventing and/or treating the disease conditions or the symptoms mediated by plasminogen activator inhibitor-1, comprising a compound described in any one of claims 1 to 4.

6. A medical drug for preventing and/or treating tissue fibrosis, comprising a compound described in any one of claims 1 to 5 and a pharmacologically acceptable carrier.

7. A medical drug for preventing and/or treating thrombosis, comprising a compound described in any one of claims 1 to 5 and a pharmacologically acceptable carrier.

## Amended claims

### Amended claims under Art. 19.1 PCT

**1.** A 5-phenyl-3-pyrazolone derivative represented by the following formula (I): 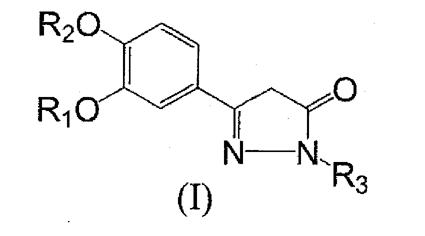 wherein R₁ represents a C₁ to C₈ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or condensed polycyclic aryl group; or a monocyclic or condensed polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more of said substituents, or an indanyl group; R₂ represents a C₁ to C₄ alkyl group, a C₁ to C₄ haloalkyl group, or a C₃ to C₇ cycloalkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more of an alkyl group on the nitrogen atom; an alkoxy group; an alkoxycarbonyl group; an acyl group; a monocyclic or condensed polycyclic aryl group; or a monocyclic or condensed polycyclic hetero ring having one or more hetero atoms); and R₃ represents a hydrogen atom or a C₁ to C₅ alkyl group which may have one or more substituents (a halogen atom; a hydroxyl group; a nitro group; a cyano group; an amino group which may have one or more C₁ to C₄ alkyl or benzyl groups on the nitrogen atom; a carboxyl group; an alkyl group; a cycloalkyl group which may have one or more phenyl or alkyl groups; a haloalkyl group; a carbamoyl group which may have one or more alkyl groups on the nitrogen atom; an alkoxy group; an alkoxycarbonyl group; an acyl group; a monocyclic or condensed polycyclic aryl group; or a monocyclic or condensed polycyclic hetero ring having one or more hetero atoms), a C₃ to C₇ cycloalkyl group which may have one or more of said substituents, a 3 to 7-membered monocyclic nonaromatic hetero ring with one or more hetero atoms which may have one or more substituents (a methyl group; a hydroxyl group; or a carboxyl group), a monocyclic or condensed polycyclic aryl group which may have one or more substituents (a methyl group; a hydroxyl group; or a carboxyl group), or a monocyclic or condensed polycyclic hetero aryl group having one or more hetero atoms; or an optical isomer, a pharmaceutically acceptable salt thereof, or a hydrate or solvate thereof, provided that 4-[3-(3,4-dimethoxyphenyl)-5-oxo-4,5-dihydropyrazol-1-yl] benzoic acid and 5-(3,4-dimethoxyphenyl)-2,4-dihydropyrazol-3-one are eliminated.

**2.** A compound described in claim 1, wherein R₁ represents a C₁ to C₈ alkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to acyl group; a 6 to 10-membered monocyclic or condensed polycyclic aryl group; a 3 to 10-membered monocyclic or condensed polycyclic nonaromatic hetero ring with one or more hetero atoms; or a 5 to 10-membered monocyclic or condensed polycyclic hetero aryl group with one or more hetero atoms), a C₃ to C₇ cycloalkyl group which may have one or more of said substituents, a 3 to 7-membered monocyclic nonaromatic hetero ring with one or more hetero atoms which may have one or more of said substituents, or an indanyl group; R₂ represents a C₁ to C₄ alkyl group, a C₁ to C₄ haloalkyl group, or a C₃ to C₇ cycloalkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to C₆ acyl group; a 6 to 10-membered monocyclic or condensed polycyclic aryl group; a 3 to 10-membered monocyclic or condensed polycyclic nonaromatic hetero ring with one or more hetero atoms; or a 5 to 10-membered monocyclic or condensed polycyclic hetero aryl group with one or more hetero atoms); and R₃ represents a hydrogen atom, a C₁ to C₅ alkyl group which may have one or more substituents (fluorine; chlorine; bromine; iodine; a hydroxyl group; a nitro group; a cyano group; an amino group which may have one or more C₁ to C₄ alkyl or benzyl groups on the nitrogen atom; a carboxyl group; a C₁ to C₆ alkyl group; a C₃ to C₇ cycloalkyl group which may have one or more phenyl or C₁ to C₆ alkyl groups; a C₁ to C₆ haloalkyl group; a carbamoyl group which may have one or more C₁ to C₄ alkyl groups on the nitrogen atom; a C₁ to C₆ alkoxy group; a C₁ to C₆ alkoxycarbonyl group; a C₁ to C₆ acyl group; a 6 to 10-membered monocyclic or condensed polycyclic aryl group; a 3 to 10-membered monocyclic or condensed polycyclic nonaromatic hetero ring with one or more hetero atoms; or a 5 to 10-membered monocyclic or condensed polycyclic hetero aryl group with one or more hetero atoms), a C₃ to C₇ cycloalkyl group which may have one or more of said substituents, a 3 to 7-membered monocyclic nonaromatic hetero ring with one or more hetero atoms which may have one or more of said substituents, a 6 to 10-membered monocyclic or condensed polycyclic aryl group which may have one or more substituents (a methyl group; a hydroxyl group; or a carboxyl group), or a 5 to 10-membered monocyclic or condensed polycyclic heteroaryl group having one or more hetero atoms, provided that 4-[3-(3,4-dimethoxyphenyl)-5-oxo-4,5-dihydropyrazol-1-yl] benzoic acid and 5-(3,4-dimethoxyphenyl)-2,4-dihydropyrazol-3-one are eliminated.

**3.** A compound described in claim 1 or 2, wherein R₁ is a C₁ to C₈ alkyl group, a C₁ to C₅ alkyl group having one or more phenyl or furyl groups as substituents, a C₁ to C₅ alkyl group with one or more C₃ to C₇ cycloalkyl groups, as substituents, which may have one or more phenyl or C₁ to C₃ alkyl groups as substituents, a C₄ to C₆ cycloalkyl group which may have one or more substituents (a methyl group; hydroxyl group; a carboxyl group), a 5 to 6-membered monocyclic nonaromatic hetero ring with one or more hetero atoms which may have one or more substituents (a methyl group; hydroxyl group; a carboxyl group), or an indanyl group; R₂ is a methyl group, an ethyl group, a trifluoromethyl group, a difluoromethyl group, a fluoromethyl group, or a C₄ to C₆ cycloalkyl group having one or more substituents (a methyl group; a hydroxyl group; a carboxyl group); and R₃ is a hydrogen atom, a C₁ to C₃ alkyl group which may have a substituent (a C₁ to C₃ alkyl group; a carboxyl group; an amino group which may have one or more C₁ to C₄ alkyl or benzyl groups on the nitrogen atom; a piperidine group which may have one or more C₁ to C₄ alkyl or benzyl groups on the nitrogen atom; a phenyl group), a phenyl group which may have one or more substituents (a methyl group; a hydroxyl group; a carboxyl group), a C₄ to C₆ cycloalkyl group which may have one or more substituents (a methyl group; a hydroxyl group; a carboxyl group), provided that 4-[3-(3,4-dimethoxyphenyl)-5-oxo-4,5-dihydropyrazol-1-yl] benzoic acid and 5-(3,4-dimethoxyphenyl)-2,4-dihydropyrazol-3-one are eliminated.

**4.** A compound described in any one of claims 1 to 3, wherein R₁ is a methyl group, an ethyl group, a 3-pentyl group, a C₁ to C₅ alkyl group having a phenyl group as a substituent, a cyclopentyl group, a cyclopropylmethyl group, a (1-methylcyclopropyl) methyl group, a (1-phenylcyclopropyl) methyl group, a 3-tetrahydrofuryl group, a 2-ethylbutyl group, or an 2-indanyl group; R₂ is a methyl group, an ethyl group, a difluoromethyl group, or a cyclopentyl group; and R₃ is a hydrogen atom, a methyl group, an ethyl group, a cyclopentyl group, a phenyl group, a 4-hydroxycarbonylphenyl group, a benzyl group, a phenethyl group, a 2-(dibenzylamino)ethyl group, a hydroxycarbonylmethyl group, or a (1-methylpiperidine-3-yl)methyl group, provided that 4-[3-(3,4-dimethoxyphenyl)-5-oxo-4,5-dihydropyrazol-1-yl] benzoic acid and 5-(3,4-dimethoxyphenyl)-2,4-dihydropyrazol-3-one are eliminated.

**5.** A medical drug for preventing and/or treating the disease conditions or the symptoms mediated by plasminogen activator inhibitor-1, comprising a compound described in any one of claims 1 to 4.

**6.** A medical drug for preventing and/or treating tissue fibrosis, comprising a compound described in any one of claims 1 to 5 and a pharmacologically acceptable carrier.

**7.** A medical drug for preventing and/or treating thrombosis, comprising a compound described in any one of claims 1 to 5 and a pharmacologically acceptable carrier.

Statement under Art. 19.1 PCT
The claims described in the replacement sheet are connected with the originally submitted claims as follows.
Claims 1 to 4 were replaced by the amended claims assigned the same numbers.
Claims 5 to 7 remain unchanged.
   The compound 4-[3-(3,4-dimethoxyphenyl)-5-oxo-4,5-dihydropyrazol-1-yl] benzoic acid described in Reference Example 4 of Reference 1 (WO 2006/129587 A1) and the compound 5-(3,4-dimethoxyphenyl)-2,4-dihydropyrazol-3-one described in the EXAMPLE 9 of Reference 2 (WO 2003/011287 A1) cited in the international search report were excluded from the inventions according to claims 1 to 4.
   Due to the amendment, the inventions according to claims 1 to 4 have novelty.
   Reference 1 does not suggest at all that the compound which is an intermediate of the final compound described in Reference example 4 or the like has an activity similar to that of the final compound which is considered effective for lung fibrosis and a plasminogen production inhibitory activity, and such activities cannot be anticipated by a person skilled in the art.
   Reference 2 does not describe that the compound described in EXAMPLE 9 or the like has a biological activity, and furthermore, it does not suggest at all that the final compound obtained using the compound described in EXAMPLE 9 has a pharmacological activity involved in lung fibrosis and a plasminogen production inhibitory activity, and such activities cannot be anticipated by a person skilled in the art.
   Consequently, the inventions according to amended claims 1 to 4 have inventive step over References 1 and 2.
